# EUROPEAN PATENT APPLICATION

(11) **EP 2 278 014 A2**
(43) Date of publication of application: **26.01.2011**
(21) Application number: 10177975.9
(22) Date of filing: 04.02.2005
(51) Int. Cl.: C12N 15/53, C07C 55/08, C12P 7/04, C12P 7/40, C12P 7/44, C07C 51/08, C07C 51/09, C07C 51/38, C07C 53/126, C07C 55/02, C07C 51/353, C07C 253/30, C07C 303/28, C07C 67/343, C07C 69/34

(54) **Method for producing alcohol and carboxylic acid having optical activity**

(30) Priority: 04.02.2004 JP 2004027815; 13.04.2004 JP 2004147023
(62) Divisional of application: 05710145.3
(71) Applicant: API Corporation, Osaka 541-0046 (JP)
(72) Inventor: Dekishima, Yasumasa, Kanagawa Kanagawa 2270033 (JP); Kawabata, Hiroshi, Kanagawa Kanagawa 2270033 (JP); Hiraoka, Hirotoshi, Kanagawa Kanagawa 2270033 (JP); Ueda, Makoto, Kanagawa Kanagawa 2270033 (JP); Uehara, Hisatoshi, Kanagawa Kanagawa 2270033 (JP)
(74) Representative: Polypatent

(57) **Abstract**

The present invention provides a method for producing (R)- or (S)-3-methyl carboxylic acid represented by the following formula (5): 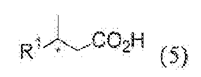 wherein R¹ represents an alkyl group containing 3 to 5 carbon atoms, and * represents an asymmetric carbon,
which comprises decarboxylating (R)- or (S)-1-methylalkyl malonic acid having optical activity represented by the following formula (1) in the presence of a highly polar solvent and/or an additive for promoting decarboxylation: 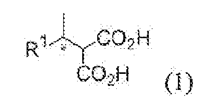 wherein R¹ has the same definition as described above, and * represents an asymmetric carbon.

## Description

### TECHNICAL FIELD

The present invention relates to a method of producing (S)-2-pentanol or (S)-2-hexanol which are industrially useful compounds as intermediate raw materials for pharmaceuticals, agrichemicals, etc, which comprises allowing microorganisms belonging to genus Issatchenkia or the like, a product obtained by treating the above microorganisms, and/or a culture obtained by culturing the above microorganism, to act on 2-pentanone or 2-hexanone. In addition, the present invention also relates to a method of producing (S)-2-pentanol or (S)-2-hexanol which comprises allowing transformed cells wherein DNA encoding a protein (carbonyl reductase) having ability to reduce a carbonyl group to synthesize optically active alcohol, which is obtained from the above microorganisms, has been expressed, a product obtained by treating the above cells, and/or a culture solution of the above cells, to act on 2-pentanone or 2-hexanone. Moreover, the present invention also relates to optically active 1-methylalkyl malonic acid, a production method thereof, and a method for producing optically active 3-methyl carboxylic acid.

### BACKGROUND ART

As a method for chemically producing (S)-2-pentanol or (S)-2-hexanol, a method involving reduction of 2-pentanone in the presence of a dendrimer of polyamide amine and gluconolactone (J. Am. Chem. Soc., Vol. 123, pp. 5956-5961, 2001), or a method involving reduction of 2-hexanone using optically active boron (Japanese Patent Application Laid-Open No. 11-240894), has been known, for example. However, these methods have not provided a satisfactory optical purity of a product.

On the other hand, as a method for generating an optically active alcoholic body using the cell mass of microorganisms and/or a product obtained by treating the above cell mass, a method of the optically selective hydrolysis of an ester by using microorganisms to act on a racemic body ester compound, so as to generate an optically active alcoholic body, has been known (Japanese Patent Application Laid-Open No. 10-4998). However, this method has been problematic in terms of low yield, and in that alcohol or an ester thereof having undesired stereochemistry should be discarded. In addition, as a method of stereoselectively reducing a compound having a keto group to generate an optically active alcoholic body, a method for producing an optically active alcoholic body by allowing microorganisms to act on 2-pentanone or 2-hexanone has been known (Tetrahedron: Asymmetry, vol. 14, pp. 2659-2681, 2003). However, this method has also been problematic in that the optical purity or production level of a product is not satisfactory, in that the method comprises a complicated pretreatment of a cell mass used in the reaction such as immobilization of the cell mass or acetone treatment, and also in that the concentration of a substrate added is low. Thus, this method has not been practical.

Optically active 1-methylalkyl malonic acid has been known to be a compound useful as a pharmaceutical or agrichemical intermediate. Moreover, optically active 3-methyl carboxylic acid has also been known to be a compound useful as a pharmaceutical or agrichemical intermediate.

Optimally active 1-methylbutyl malonic acid is a compound useful as an intermediate of a barbituric acid derivative which exhibits neurodepressive action (refer to International Publication WO00/24358, for example). In addition, optically active 3-methyl hexanoic acid and optically active 3-methyl heptanoic acid, which can be synthesized from optically active 1-methyl-alkyl malonic acid, are used as pharmaceutical intermediates of prostaglandins or the like (Japanese Patent Application Laid-Open No. 62-265279, for example).

The synthesis of (S)-1-methylbutyl malonic acid using optically active 2-pentanol has been reported (J. Am. Chem. Soc., 1950, 72, 4695). However, in order to obtain Optically active 2-pentanol, this method involves an extremely complicated method, which comprises converting racemic body 2-pentanol to a phthalate monoester and resolving it with brucine, followed by hydrolysis. In addition, since the above compound is resolved, the rest of the material be used. Further, the molecular weights of the auxiliary group and the resolving agent are relatively large based on that of alcohol to be optically resolved, and thus this method is inefficient.

Several methods for synthesizing 3-methyl carboxylic acid via a decarboxylation reaction of 1-methylalkyl malonic acid have been know (refer to J. Am. Chem. Soc., 1950, 72, 4695, and Nouveau Journal de Chime, 1985, 9, 557, for example), However, since all of these methods are collectively addition methods using no solvents, causing difficult reaction control, and further require a high temperature (180°C), the industrial application of these methods is difficult.

A method of using an additive to a substrate that is different from that of the present invention so as to carry decarboxylation at a low temperature has also been known. It was examined whether or not a method involving reflux in acetonitrile in the presence of copper oxide (refer to J. Am. Chem. Soc., 1993, 115, 801, for example) or a method involving heating in the presence of sulfuric acid (refer to Org. Lett., 2002, 4, 1571, for example) can be applied to the substrate of the present invention. As a result, it was found that a significant effect of accelerating the reaction was not observed. From such a result, it was revealed that the method of using an additive to carry out a decarboxylation reaction at a low temperature can be applied to some compounds, but cannot be applied to the other compounds.

On the other hand, a method of carrying out a reaction in a solvent at a temperature between approximately 100°C and 150°C has also been known (refer to J. Org. Chem., 1993, 48, 2994, for example). However, there have been no reports regarding such a solvent effect. Further, since the reaction temperature required during decarboxylation is different depending on the type of a substrate, the effect of carrying out a decarboxylation reaction at a low temperature in a solvent has not been clarified.

In addition, these methods have never been considered from the viewpoint of safety such as the control of carbon dioxide generated. Thus, problems regarding industrial application of such a decarboxylation reaction still have remained.

As a method for synthesizing an optically active 1-methylbutyl masonic ester that is considered to be a synthetic precursor of optically active 1-methylbutyl malonic acid, a synthetic method involving induction from citronellol has been known (refer to International Publication WO00/24358, for example). However, this method has been problematic in that it is a multi-step method, which brings on a low yield. In addition, as a method for synthesizing an optically active 1-methylpenyl malonic ester, a synthetic method involving an asymmetric 1,4-addition reaction has been known (refer to Tetrahedron Asym., 2001, 12, 1151, for example). However, since a sufficient optical purity cannot be obtained (50%ee at the maximum), this method is not practical.

As a method for synthesizing optically active 3-methyl hexanoic acid or optically active 3-methyl heptanoic acid, which can be synthesized from optically active 1-methylalkyl malonic acid, an addition reaction of an organic copper reagent to a crotonic acid derivative having an asymmetric auxiliary group has been known (refer to Helv. Chim. Acta., 1985, 68, 212, for example). However, this method requires introduction of an expensive asymmetric auxiliary group into a molecule, and further, it is necessary to use an equivalent amount of organic copper reagent causing a problem regarding a waste liquid treatment. Thus, it cannot be said that this method is industrially applicable. Moreover, optical resolution of a racemic compound has also been known (refer to Japanese Patent Application Laid-Open No. 62-265279, for example). However, since compounds with desired stereochemistry can be obtained only at 50% at the maximum by such resolution, this method brings on poor efficiency. Moreover, a moiety of compounds with undesired steric configuration is discarded, resulting in an enormous environmental burden. Furthermore, a method involving induction from citronellic acid or the like has also been known (refer to US Patent No. 5136020, and Tetrahedron, 1977, 33, 289, for example), but this method has been problematic in terms of multi-step procedure and low yield.

Still further, there has been a method for synthesizing optically active 1-methylbutyl malonic acid (refer to J. Am. Chem. Soc., 1950, 72, 4695). However, regarding this method, it is described that optical purity is significantly decreased during the coupling with a malonic ester following bromination, and that thereafter, even if it is induced to optically active 1-methylbutyl malonic acid and crystallization is then repeatedly carried out, such an optical purity is increased only up to approximately 70%ee. That is to say, optically active 1-methylbutyl malonic acid with high optical purity required as a pharmaceutical or agrichemical intermediate cannot be synthesized by the conventional methods, and thus, it has been desired that a method for synthesizing the above compound without decreasing optical purity be developed.

Further, examples of the synthesis of optically active 1-methylheptyl masonic acid (refer to Nouveau Journal de Chime, 1985, 9, 557) and optically active 1-methylpropyl masonic acid labeled with a radioactive element (refer to J. Am. Chem. Soc., 1980, 102, 7344) have been known. However, these methods are significantly disadvantageous regarding industrial application in that a long period of time (over 12 hours) is required during the reaction with a malonic ester, in that a large amount of solvent (50 times volume) is required during recrystallization of dicarboxylic acid, in that a high temperature (180°C) is required in a decarboxylation reaction, and in that these methods require high costs and are not efficient. Moreover, the optical purities of such compounds have been reported only in terms of optical rotation, and thus a problem that precise optical purity is unknown has remained.

### DISCLOSURE OF THE INVENTION

It is an object of the present invention to provide a novel method for industrially simply and inexpensively producing (S)-2-pentanol or (S)-2-bexanol having higher optical purity, and preferably (S)-2-pentanol or (S)-2-hexanol having an optical purity of 99.0%ee or greater. It is another object of the present invention to provide an industrial production method for inexpensively and efficiently producing optically active 1-methylalkyl masonic acid and optically active 3-methyl carboxylic acid at high optical purity.

As a result of intensive studies regarding a production method of (S)-2-pentanol or (S)-2-hexanol, which are directed towards achieving the aforementioned objects, the present inventors have found that using a certain type of microorganisms belonging to genus Brettanomyces or the like, (S)-2-pentmol or (S)-2-hexanol can be simply and efficiently generated using 2-pentanone or 2-hexanone as a substrate. In addition, the present inventors have isolated carbonyl reductase which reduces 2-pentanone or 2-hexanone to generate (S)-2-pentanol or (S)-2-hexanol and DNA encoding the above enzyme from microorganisms belonging to genus Issatchenkia, which are one type of the aforementioned microorganisms, and have analyzed the nucleotide sequence thereof. Moreover, the present inventors have found that a product of interest, namely, (S)-2-pentanol or (S)-2-hexanol can be obtained at high optical purity and at a high concentration by producing a transformant wherein the above DNA has been allowed to express, and then allowing the transformed cells, a product obtained by treating the above cells, and/or a culture solution of the above cells, to act on 2-pentanone or 2-hexanone used as a raw material.

Furthermore, the present inventors have also found that a substitution reaction can be carried out while maintaining high optical purity by converting optically active alcohol to a leaving group and then treating the resulting compound with a carbon nucleophile, and that the obtained optically active compound is hydrolyzed and then crystallized, so as to efficiently produce optically active 1-methylatkvl malonic acid art high optical purity. Still further, the present inventors have established an industrially simple and excellent production method, wherein a highly-polar solvent and/or an additive for promoting decarboxylation is used when optically active 1-methylakyl malonic acid is converted to optically active 3-methyl carboxylic acid by decarboxylation, so that the reaction can be carried out under conditions that are much more moderate than those of the conventional method, and so that generation of carbon dioxide can be controlled.

The present invention has been completed based on these findings,

That is to say, the pre-sent invention provides the following features of the invention:
(1) A method of producing (S)-2-pentanol which comprises allowing microorganisms or transformed cells, a product obtained by treating said microorganisms or cells, a culture solution of said microorganisms or cells, and/or a crude purified product or purified product of a carboxyl reductase fraction obtained from said microorganisms or cells, to act on 2-pentanone, wherein when a fresh cell mass of said microorganisms or transformed cells, which has not been pretreated with a solvent, is allowed to act on 2-pentanone, (S)-2-pentanol having an optical purity of 95% e.e. or greater can be generated, and the productivity thereof is 1 mg or more of (S)-2-pentanol/g of dry cell mass weight/hour.
(2) A method of producing (S)-2-hexanol which comprises allowing microorganisms or transformed cells, a product obtained by treating said microorganisms or cells, a culture solution of said microorganisms or cells, and/or a crude purified product or purified product of a carbonyl reductase fraction obtained from said microorganisms or cells, to act on 2-hexanone, wherein when a fresh cell mass of said microorganisms or transformed cells, which has not been pretreated with a solvent, is allowed to act on 2-hexanone. (S)-2-hexanol having an optical purity of 95% e.e. or greater can be generated, and the productivity thereof is 1 mg or more of (S)-2-hexanol/g of dry cell mass weight/hour.
(3) A method for producing (S)-2-pentanol or (S)-2-hexanol having high optical purity, wherein microorganisms selected from the group consisting of genus Brettanomyces, genus Candida, genus Hortaea, genus Issatchenkia, genus Lodderomyces, genus Pichia, genus Rhodotorula, genus Arthrobacter, genus Brevibacterium, genus Crutobacterium, genus Geobacillus, genus Microbacterium, genus Ocurobactrum, genus Paracoccus, genus Rhizobium, and genus Rhodococcus, a product obtained by treating said microorganisms, a culture solution of said microorganisms, and/or a crude purified product or purified product of a carbonyl reductase fraction obtained from said microorganisms, are allowed to act on 2-pentanone or 2-hexanone, so as to generate (S)-2-pentanol or (S)-2-hexanol.
(4) A method for producing (S)-2-pentanol or (S)-2-hexanol having high optical purity, wherein transformed cells wherein DNA encoding carbonyl reductase obtained from microorganisms selected from the group consisting of genus Brettanomyces, genus Candida, genus Hortaea, genus Issatchenkia, genus Lodderomyces, genus Pichia, genus Rhodotorula, genus Arthrobacter, genus Brevibacterium, genus Crutobacterium, genus Geobacillus, genus Microbacterium, genus Ochrobactrum, genus Paracoccus, genus Rhizobium, and genus Rhodococcus, has been allowed to express, a product obtained by treating said cells, a culture solution of said cells, and/or a crude purified product or purified product of a carbonyl reductase fraction obtained from said cells, are allowed to act on 2-pentanone or 2-hexanone, so as to generate (S)-2-pentanol or (S)-2-hexanol.
(5) The production method according to (3) or (4) above, wherein the microorganisms are selected from the group consisting of *Brettanomyces bruxellensis, Brettanomyces anomalus, Candida famala, Candida krusei, Candida maltosa, Candida tropicalis, Candida zeylanoides, Hortaea werneckii, Issatchenkia scutulata, Lodderomyces elongisporus, Pichia angusta, Pichia besseyi, Pichia cactophila, Pichia segobiensis, Pichia spartinae, Pichia trehalophila, Rhodotorula minuta, Arthrobacter oxydans, Arthrobacter polychromogenes,* Arthrobacter sp., *Arthrobacter sulfurous, Brevibacterium butanicum, Curtobacterium flaccumfaciens, Geobacillus stearothermophilus, Microbacterium keralanolyticum, Microbacterium saperdae,* Microbacterium sp., *Microbacterium testaceum, Ochrobactrum anthropi,* Ochrobactrum sp. (*Pseudomonas ovalis*), *Pracoccus denitrificans, Rhizobium radiobacter,* and Rhodococcus sp. (*Corynebacterium hydrocarboclastum*).
(6) A method for producing (S)-2-pentanol or (S)-2-hexanol having high optical purity, wherein transformed cells, wherein DNA described in any one of the following (A) to (F) has been allowed to express, a product obtained by treating said cells, and/or a culture solution of said cells, are allowed to act on 2-pentanone or 2-hexanone, so as to generate (S)-2-pentanol or (S)-2-hexanol:
   (A) DNA encoding a protein having the amino acid sequence shown in SEQ ID NO: 1;
   (B) DNA encoding a protein, which has an amino acid sequence comprising a deletion, addition, or substitution of one or several amino acids with respect to the amino acid sequence shown in SEQ ID NO: 1, and which has ability to reduce a carbonyl group to synthesize optically active alcohol;
   (C) DNA encoding a protein, which has an amino acid sequence showing homology of 50% or more with the amino acid sequence shown in SEQ ID NO: 1, and which has ability to reduce a carbonyl group to synthesize optically active alcohol;
   (D) DNA having the nucleotide sequence shown in SEQ ID NO: 2;
   (E) DNA having a nucleotide sequence, which comprises a deletion, addition, or substitution of one or several nucleotides with respect to the nucleotide sequence shown in SEQ ID NO: 2, and which encodes a protein having ability to reduce a carbonyl group to synthesize optically active alcohol; and
   (F) DNA having a nucleotide sequence, which hybridizes with the nucleotide sequence shown in SEQ ID NO: 2 or a complementary sequence thereof under stringent conditions, and which encodes a protein having ability to reduce a carbonyl group to synthesize optically active alcohol.
(7) A method for producing (R)- or (S)-3-methyl carboxylic acid represented by the following formula (5): 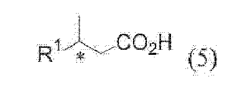 wherein R¹ represents an alkyl group containing 3 to 5 carbon atoms, and * represents an asymmetric carbon,
   which comprises decarboxylating (R)- or (S)-1-methylalkyl malonic acid having optical activity represented by the following formula (1) in the presence of a highly polar solvent and/or an additive for promoting decarboxylation: 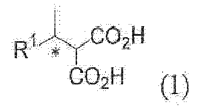 wherein R¹ has the same definition as described above, and * represents an asymmetric carbon.
(8) A method for producing (R)- or (S)-1-methylalkyl malonic acid represented by the following formula (1): 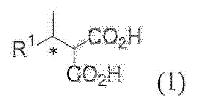 wherein R¹ represents an alkyl group containing 3 to 5 carbon atoms, and * represents an asymmetric carbon,
   which comprises allowing optically active alcohol represented by the following formula (2) to react with a sulfonylation agent: 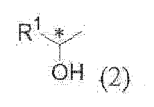 wherein R¹ has the same definition as described above, and * represents an asymmetric carbon, so as to obtain an optically active compound represented by the following formula (3): 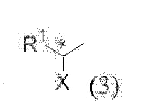 wherein R¹ has the same definition as described above, X represents a sulfonyloxy group, and * represents an asymmetric carbon;
   allowing the optically active compound to react with a carbon nucleophile represented by the following formula (9) in the presence of a base: 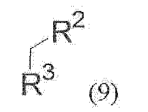 wherein each of R² and R³ independently represents an ester group, a carboxyl group, or a cyano group, wherein R² and R³ may together form a cyclic structure, so as to obtain an optically active compound represented by the following formula (4): 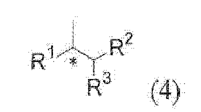 wherein R¹, R², and R³ have the same definitions as described above, and * represents an asymmetric carbon, and
   hydrolyzing the obtained optically active compound.
(9) (R)-1-methylalkyl malonic acid or (S)-1-methylalkyl masonic acid having an optical purity of 90%ce or greater, which is represented by the following formula (1): 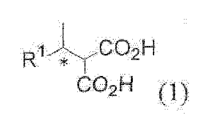 wherein R¹ represents an alkyl group containing 3 to 5 carbon atoms, and * represents an asymmetric carbon.
(10) The (R)-1-methylalkyl malonic acid or (S)-1-methylalkyl malonic acid according to (9) above, wherein R¹ represents an n-propyl group or an n-butyl group.
(11) A method for producing an optically active substance represented by the following formula (6): 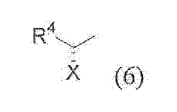 wherein R⁴ represents an n-propyl group, and X represents a sulfonyloxy group,
   which comprises: allowing microorganisms or transformed cells containing a carbonyl reductase having activity to react with 2-pentanone to generate (S)-2-pentanol, wherein it is able to generate (S)-2-pentanol having an optical purity of 95% e.e. or greater when the fresh cell mass thereof, which has not been pretreated with a solvent, is allowed to act on 2-pentanone, and the productivity thereof is 10 mg or more of (S)-2-pentanol/g of dry cell mass weight/hour, a product obtained by treating said microorganisms or cells, a culture solution of said microorganisms or cells, and/or a crude purified product or purified product of a carbonyl reductase fraction obtained from said microorganisms or cells, to act on 2-pentanone, so as to convert it to (S)-2-pentanol; and allowing the obtained (S)-2-pentanol to react with a sulfonylation agent, so as to convert it to the optically active substance represented by the above formula (6).
(12) A method for producing an optically active substance represented by the following formula (6): 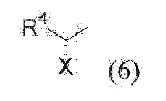 wherein R⁴ represents an n-butyl group, and X represents a sulfonyloxy group,
   which comprises: allowing microorganisms or transformed cells containing a carbonyl reductase having activity to react with 2-hexanone to generate (S)-2-hexanol, wherein it is able to generate (S)-2-hexanol having an optical purity of 95% e.e. or greater when the fresh cell mass thereof, which has not been pretreated with a solvent, is allowed to act on 2-hexanone, and the productivity thereof is 10 mg or more of (S)-2-hexanol/g of dry cell mass weight/hour, a product obtained by treating said microorganisms or cells, a culture solution of said microorganisms or cells, and/or a crude purified product or purified product of a carbonyl reductase fraction obtained from said microorganisms or cells, to act on 2-hexanone, so as to convert it to (S)-2-hexanol; and allowing the obtained (S)-2-hexanol to react with a sulfonylation agent, so as to convert it to the optically active substance represented by the above formula (6).
(13) The method according to (11) or (12) above, which further comprises a step of allowing the optically active substance represented by formula (6) to react with a carbon nucleophile represented by the following formula (9) in the presence of a base: 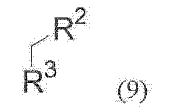 wherein each of R² and R³ independently represents an ester group, a carboxyl group, or a cyano group, wherein R² and R³ may together form a cyclic structure, so as to convert it to an optically active compound represented by the following formula (7): 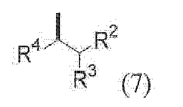 wherein R² and R³ have the same definitions as described above, and R⁴ represents an n-propyl group or an n-butyl group.
(14) A method for producing (R)-1-methylbutyl malonic acid or (R)-1-methylpentyl malonic acid, which comprises; allowing the optically active substance represented by formula (6) obtained by the method according to (11) or (12) above to react with a carbon nucleophile represented by the following formula (9) in the presence of a base: 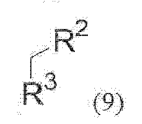 wherein each of R² and R³ independently represents an ester group, a carboxyl group, or a cyano group, wherein R² and R³ may together form a cyclic structure, so as to convert it to an optically active compound represented by the following formula (7): 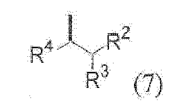 wherein R² and R³ have the same definitions as described above, and R⁴ represents an n-propyl group or an n-butyl group, and
   hydrolyzing the obtained optically active compound, so as to convert it to (R)-1-methylbutyl malonic acid or (R)-1-methylpentyl malonic acid represented by the following formula (8): 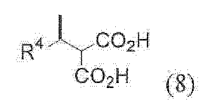 wherein R⁴ has the same definition as described above.
(15) A method for producing (R)-3-methyl hexanoic acid or (R)-3-methyl heptanoic acid, which comprises allowing the optically active substance represented by formula (6) obtained by the method according to (11) or (12) above to react with a carbon nucleophile represented by the following formula (9) in the presence of a base: 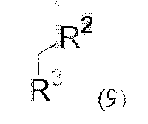 wherein each of R² and R³ independently represents an ester group, a carboxyl group, or a cyano group, wherein R² and R³ may together form a cyclic structure, so as to convert it to an optically active compound represented by the following formula (7): 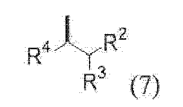 wherein R² and R³ have the same definitions as described above, and R⁴ represents an n-propyl group or an n-butyl group,
   hydrolyzing the obtained optically active compound, so as to convert it to (R)-1-methylbutyl malonic acid or (R)-1-methylpentyl malonic acid represented by the following formula (8): 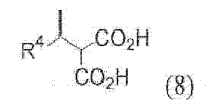 wherein R⁴ has the same definition as described above, and
   decarboxylating the obtained (R)-1-methylbutyl malonic acid or (R)-1-methylpentyl malonic acid.

### BEST MODE FOR CARRYING OUT THE INVENTION

The embodiments of the present invention will be described below. However, these contents are not intended to limit the scope of the present invention.

### 1. Method for producing optically active alcohol using microorganisms or the like

The method for producing (S)-2-pentanol or (S)-2-hexanol according to the present invention is a method of producing (S)-2-pentanol (or (S)-2-hexaol) which comprises allowing microorganisms or transformed cells, a product obtained by treating said microorganisms or cells, a culture solution of said microorganisms or cells, and/or a crude purified product or purified product of a carbonyl reductase fraction obtained from said microorganisms or cells, to act on 2-pentanone (or 2-hexanone), wherein when a fresh cell mass of said microorganisms or transformed cells, which has not been pretreated with a solvent, is allowed to act on 2-pentanone (or 2-hexanone), (S)-2-pentanol (or (S)-2-hexanol) having an optical purity of 95% e.e. or greater can be generated, and the productivity thereof is 1 mg or more of (S)-2-pentanol (or (S)-2-hexanol)/g of dry cell mass weight/hour.

It is to be noted that the terms "2-pentanone" and "2-hexanone" are used in the present specification to mean 2-pentanone and 2-hexanone having a linear carbon chain.

As stated above, microorganisms or transformed cells used for the method of the present invention are **characterized in that** when a fresh cell mass of the above microorganisms or transformed cells, which has not been pretreated with a solvent, is allowed to act on 2-pentanone (or 2-hexanone), (S)-2-pentanol (or (S)-2-hexanol) having an optical purity of 95% e.e. or greater can be generated, and the productivity thereof is 1 mg or more of (S)-2-pentanol (or (S)-2-hexanol)/g of dry cell mass weight/hour. Examples of a solvent used herein may include acetone, toluene, dimethyl sulfoxide, and 2-propanol. Examples of a ptetreatment may include immersion of a cell mass, and a method of immersing a cell mass and then drying it under reduced pressure. A method for producing (S)-2-pentanol or (S)-2-hexanol, using microorganisms, or transformed cells, which need such a pretreatment, requires man power and cost for the treatment, and it is difficult for the method to obtain reproducible results. Thus, such a method is not preferable.

The optical purity of (S)-2-pentanol or (S)-2-hexanol generated may be 95% e.e. or greater, preferably 98% e.e. or greater, and more preferably 99% e.e. or greater.

In addition, the productivity of (S)-2-pentanol be 1 mg or more of (S)-2-pentanol/g of dry cell mass weight/hour, preferable 2 mg or more of (S)-2-pentanol/g of dry cell mass weight/hour, more preferably 5 or more of (S)-2-pentanol/g of dry cell mass weight/hour, further preferably 10 mg or more of (S)-2-pentanol/g of dry cell mass weight/hour, and particularly preferably 20 mg or more of (S)-2-pentanol/g of dry cell mass weight/hour,

Moreover, the productivity of (S)-2-hexanol may be 1 mg or more of (S)-2-hexanol/g of dry cell mass weight/hour, preferably 2 mg or more of (S)-2-hexanol/g of dry cell mass weight/hour, preferably 5 mg or more of (S)-2-hexanol/g of dry cell weight/hour, further more preferably 10 mg or more of (S)-2-hexanol/g of dry cell weight/hour, further more preferably 20 mg or more of (S)-2-hexanol/g of dry cell weight/hour, further more preferably 50 mg or more of (S)-2-hexanovg of dry cell weight/hour, and particularly preferably 100 mg or more of (S)-2-hexanol/g of dry cell mass weight/hour.

As an of the method for producing (S)-2-pentanol or (S)-2-hexanol according to the present invention, a production method can be carried out, using a transformed strain wherein DNA, which encodes a protein having the amino acid sequence shown in SEQ ID NO: 1 or a protein that is a homolog of the above amino acid sequence and has ability to reduce a carbonyl group to synthesize optically active alcohol (hereinafter simply referred to as "carbonyl reductase" at times), has been allowed to express.

In the specification, carbonyl reductase activity means activity of asymmetrically reducing a carbonyl group contained in a carbonyl group-containing compound so as to convert it to optically active alcohols. Such activity can be calculated by allowing a protein of interest acting as an enzyme, a transformant having ability to express the above protein, a product obtained by treating the transformant, or a culture solution thereof, to act on a reaction solution that contains a carbonyl group-containing compound as a substrate and also contains NADPH or NADH as a coenzyme, and then measuring the reduced initial rate of NADPH or NADH in the reaction solution based on a change in the absorbance of the reaction solution.

The type of carbonyl reductase used in the present invention is not particularly limited, as long as it is an enzyme capable of generating (S)-2-pentanol or (S)-2-hexanol from 2-pentanone or 2-hexanone. In order to measure the carbonyl reductase activity of the carbonyl reductase in the present invention, a carbonyl group-containing compound is used as a substrate. As such a carbonyl group-containing compound, not only 2-pentanone or 2-hexanone, but also structurally similar compounds such as a substituted compound or derivative thereof can preferably be used. An example of such a structurally similar compound is 1-acetoxy-3-chloro-2-propanone.

The amino acid sequence of carboxyl reductase and a nucleotide sequence encoding the above amino acid sequence have been clarified by the descriptions of the present specification. Thus, as described later, using a probe produced based on a nucleotide sequence encoding a part of or the entire amino acid sequence of carbonyl reductase, DNA encoding carbonyl reductase can be isolated from any given microorganisms having carbonyl reductase activity, and the carbonyl reductase can be then obtained based on the DNA by common genetic engineering.

Moreover, as with purification conducted to complete the present invention, carbonyl reductase can be purified from microorganisms having carbonyl reductase activity, namely, microorganisms having DNA encoding carbonyl reductase, such as those selected from the group consisting of genus Brettanomyces, genus Candida, genus Hortaea, genus Issatchenkia, genus Lodderomyces, genus Pichia, genus Rhodotorula, genus Arthrobacter, genus Brevibacterium, genus Crutobacterium, genus Geobacillus, genus Microbacterium, genus Ochrobactrum, genus Paracoccus, genus Rhizobium, and genus Rhodococcus, and preferably from a culture of the yeast of genus Issatchenkia.

A preferably used yeast of genus Issatchenkia is Issatchankia scutulata var. scutulata. For example, carbonyl reductase derived from the Issatchankia scutulata var. scutulata JCM1828 strain is particularly preferably used as the carbonyl reductase of the present invention because it is excellent in terms of production of optically active alcohol. This cell strain can be obtained from Japan Collection of Microorganism (JCM), RIKEN Bio Resource Center.

As carbonyl reductase or DNA encoding the above enzyme which is used to produce (S)-2-pentanol by allowing them to act on 2-pentanone, those derived from microorganisms belonging to genus Brettanomyces, genus Candida, genus Hortaea, genus Lodderomyces, or genus Pichia, can preferably be used.

More preferably, those derived from *Brettanomyce bruxellensis, Candida tropicalis*, *Candida zeylanoides*, *Hortaea werneckii*, *Lodderomyces elongisporus*, *Pichia segobiensis*, *Pichia spartinae*, *Arthrobacter globiformis*, *Arthrobacter oxydans*, *Arthrobacter polychromogenes*, *Curtobacterium flaccumfaciens*, *Geobacillus stearothermophilus*, *Microbacterium testaceum*, *Ochrobactrum anthropi*, Ochrobactrum sp. (*Pseudomonas ovalis*), or *Rhizobium radiobacter*, can be used.

Specifically, those derived from the following stains can particularly preferably be used: *Brettanomyces bruxellensis* NBRC 0629, *Brettanomyces bruxellensis* NARC 0797, *Candida tropicalis* NBRC 0006, *Candida zeylanoides* CBS 6408, *Candida zeylanoides* JCM 1627, *Hortaea werneckii* NBRC 4875, *Lodderomyces elongisporus* NBRC 1676, *Pichia segobiensis* JCM 10740, *Pichia spartinae* JCM 10741, *Arthrobacter globiformis* NBRC 12137, *Arthrobacter oxydans* DSM 20120, *Arthrobacter polychromogenes* DSM 342, *Curtobaeterium flaccumfaciens* ATCC 12813, *Geobacillus stearothermophilus* NBRC 12550, *Geobacillus stearothermophilus* IAM 11002, *Geobacillus stearothermophilus* IAM 11004, *Geobacillus stearothermophilus* IAM 12043, *Microbacterium testaceum* JCM 1353, *Ochrobactrum anthropi* ATCC 49237, Ochrobactrum sp. (*Pseudomonas ovalis*) NBRC 12950, Ochrobactrum sp. (*Pseudomonas ovalis*) NBRC 12952, Ochrobactrum sp. (*Pseudomonas ovalis*) NBRC 12953, and *Rhizobium radiobacter* IAM 12048.

In addition, as carbonyl reductase or DNA encoding the above enzyme which is used to produce (S)-2-hexanol by allowing them to act on 2-hexanone, those derived from microorganisms, belonging to genus Brettanomyces, genus Candida, genus Issatchenkia, genus Lodderomyces, genus Pichia, or genus Rhodotorula, can preferable be used.

Of these, those derived from *Brettanomyces anomala, Candida famata, Candida krusei*, *Candida maltosa*, *Candida zeylanoides*, *Issatchenkia scutulata*, *Lodderomyces elongisporus*, *Pichia angusta*, *Pichia cactophila*, *Pichia segobiensis*, *Pichia trehalophila*, and *Rhodotorula minuta*, can particularly preferably be used.

Specifically, those derived from the following stains can particularly preferably be used: *Brettanomyces anomala* NBRC 0627, *Candida famata* ATCC 10539, *Candida krusei* NBRC 1664, *Candida krusei* JCM 2284, *Candida krusei* JCM 2341, *Candida maltosa* NBRC 1977, *Candida zeylanoides* CBS 6408, Issatchenkia scutulata var. scutulata JCM 1828, *Lodderomyces elongisporus* NBRC 1676, *Pichia angusta* NBRC 1024, *Pichia angusta* NBRC 1071, *Pichia cactophila*, JCM 1830, *Pichia segobiensis* JCM 10740, *Pichiatrehalophila* JCM 3651, *Rhodotorula minuta* NBRC 0879, Arthrobacter sp. DSM 20407, *Arthrobacter sulfurous* (*Brevibacterium sulfureum*) JCM 1338, *Brevibacterium butanicum* ATCC 21196, *Brevibacterium sulfureum* JCM 1485, *Curtobacierium flaceunmfaciens* ATCC 12813, *Microbacterium keratanolyticum* NBRC 13309, *Microbacierium saperdae* JCM 1352, Microbacterium sp, NBRC 15615, *Ochrobactrum anthropi* ATCC 49237, Ochrobactrum sp. (*Pseudonionas ovalis*) NBRC 12952, Ochrobactrum sp. (*Pseudomonas ovalis*) NBRC 12953, *Paracoccus denitrificans* NBRC 12442, *Rhizobium radiobacter* IAM 12048, *Rhizobium radiobacter* IAM 13129, and Rhodococcus sp. ATCC 15960.

As a method of obtaining carbonyl reductase from a culture of microorganism, a common enzyme purification method can be used. For example, carbonyl reductase can be obtained by the following method. The aforementioned microorganisms are cultured in a medium commonly used in the culture of yeast, such as YM medium, so that they are allowed to sufficiently grow. Thereafter, the microorganisms are recovered, and are then disintegrated in a buffer solution, to which a reducing agent such as DTT (dithiothreitol) or a protease inhibitor such as phenylmethansulfonyl fluoride (PMSF) has been added, so as to obtain a cell-free extract. From such a cell-free extract, carbonyl reductase can be purified by the combined use of fractionation due to the solubility of a protein (precipitation in an organic solvent, salting-out with ammonium sulfate, etc.), cation exchange chromatography, anion exchange chromatography, gel filtration chromatography, hydrophobic chromatography, hydroxyapatite chromatography, affinity chromatography using chelate, pigment, antibody, etc., as appropriate.

For example, as described in the examples of the present specification, carboxyl reductase can be purified to obtain almost a single band in an electrophoresis via anion exchange chromatography using DEAE Sepharose Fast Flow (manufactured by Amersham Biosciences), hydrophobic interaction chromatography using Butyl Sepharose 4 Fast Flow (manufactured by Amersham Biosciences), anion exchange chromatography using MonoQ (manufactured by Amersham Biosciences), gel filtration chromatography using Superdex 200 (manufactured by Amersham Biosciences), etc.

As a result of sodium dodecyl sulfate-polyacrylamide gel electrophoresis (hereinafter abbreviated as SDS-PAGE), it was found that the thus purified carbonyl reductase derived from the Issatchaukia scutulata var. scutulata JCM1828 strain (hereinafter, this enzyme is referred to as "IsADH1" at times) consisted of a single type of subunit having a molecular weight of approximately 40,000 Da, and that the molecular weight thereof determined by gel filtration using Superdex 200 HR10/30 (manufactured by Amersham Biosciences) was approximately 40,000 Da. From these results, it is assumed that IsADH1 is a monomer consisting of a single type of subunit with approximately 40,000 Da.

DNA encoding carbonyl reductase can be isolated by the following method, for example.

First, carbonyl reductase is purified by the aforementioned method or the like, and the N-terminal amino acid sequence thereof is then analyzed. Thereafter, it is cleaved with an enzyme such as lysyl endopeptidase or V8 protease, and peptide fragments are then purified by reverse phase liquid chromatography or the like. Thereafter, amino acid sequences are analyzed with a protein sequencer, so as to determine several amino acid sequences.

Primers used for PCR are designed based on the determined amino acid sequences. Using the chromosomal DNA of a carbonyl reductase-producing microorganism strain or a cDNA library as a template, PCR is carried out with the PCR primers designed from the amino acid sequences, so as to obtain a portion of the DNA of the present invention, Thereafter, the obtained DNA fragment is used as a probe, and the restriction enzyme digestion product of the chromosomal DNA of the carbonyl reductase-producing microorganism strain is introduced into a phage, plasmid, or the like. A library obtained by transformation of *Escherichia coli* or a cDNA library is used to carry out colony hybridization, plaque hybridization, or the like, so as to obtain DNA encoding carbonyl reductase.

Moreover, it is also possible to obtain the DNA of the present invention by analyzing the nucleotide sequence of a DNA fragment obtained by PCR, designing PCR primers for extending to the outside of the known DNA, based on the obtained sequence, and then applying the RACE (Rapid amplification of cDNA ends) method using the cDNA of the carbonyl reductase-producing microorganism strain (Molecular Cloning, 3rd Ed., Cold Spring Harbor Laboratory Press; hereinafter referred to as Molecular Cloning).

The nucleotide sequence of the DNA encoding carbonyl reductase IsADH1, which is isolated from the chromosomal DNA of the Issatchankia scutulata var. scutulata JCM 1828 strain as described above, is as shown in SEQ ID NO: 2.

The DNA encoding carbonyl reductase IsADH1 can be genomic DNA or cDNA which is cloned by the aforementioned method. Otherwise, since the nucleotide sequence of the DNA has been clarified as described in the present specification, it can also be obtained by chemical synthesis based on the nucleotide sequence shown in SEQ ID NO: 2.

A homolog of DNA encoding IsADH1 has an amino acid sequence comprising a deletion, substitution, or addition of one or several amino acids with respect to the amino acid sequence shown in SEQ ID NO: 1, within the range that does not impair carbonyl group reductase activity. Herein, the number of amino acids to be deleted, substituted, or added, is specifically 20 or less, preferably 10 or less, and more preferably 5 or less.

In addition, the homology of IsADH1 means a protein having homology of at least 50% or more, preferably 70% or more, and more preferably 80% or more, with the amino acid sequence shown in SEQ ID NO:1,

The homology search of the aforementioned protein can be carried out on the DNA Databank of JAPAN (DDBJ) or the like as a target, using program such as FASTA or BLAST. As a result of the homology search of the amino acid sequence shown in SEQ ID NO: I performed on DDBJ as a target using BLAST program, it was found that among the known proteins, a protein showing the highest homology the Ydr541cp protein (SEQ ID NO: 3; Accession No. AAB64983) derived from *Saccharomyces cerevisiae*, whose functions are unknown, and that it showed homology of 42%.

Moreover, DNA encoding IsADH1 is DNA encoding the aforementioned IsADH1 or a homolog thereof, which encodes a protein having carbonyl reductase activity.

An example of the DNA encoding the aforementioned protein is DNA having the nucleotide sequence shown in SEQ ID NO: 2.

A homolog of the DNA encoding IsADH1 includes DNA encoding a protein having an amino acid sequence comprising a deletion, substitution, or addition of one or several amino acids with respect to the amino acid sequence shown in SEQ ID NO: 1 within the range that does not impair carbonyl group reductase activity. Herein, the number of amino acids to be deleted, substituted, or added, is specifically 60 or less, preferably 30 or less, and more preferably 10 or less.

Persons skilled in the art can appropriately introduce a substitution, deletion, insertion, and/or addition mutation into the DNA shown in SEQ ID NO: 2 by site-directed mutagenesis (Nucleic Acids Res., Vol. 10, p. 6487 (1982); Methods in Enzymol., Vol. 100, p. 448 (1983); Molecular Cloning, PCR - A Practical Approach, IRL Press, p. 200 (1991)) or other techniques, thereby obtaining a homolog of the DNA encoding IsADH1.

It is also possible that homology search be performed on database such as the DNA Databank of JAPAN (DDBJ) on the basis of the amino acid sequence of IsADH1 or a portion thereof, or DNA encoding IsADH1 or a portion thereof, so as to obtain the nucleotide sequence information of a DNA homolog encoding the protein of the present invention. Based on the nucleotide sequence information, persons skilled in the art are able to obtain the above DNA fragment from the deposited cell strains by PCR or the like.

Furthermore, a homolog of the DNA encoding IsADH1 can also be obtained by performing hybridization, such as the colony hybridization method, plaque hybridization method, or Southern blot hybridization method, on DNA prepared from any given microorganisms having carbonyl reductase activity under stringent conditions using DNA encoding IsADH1 or a portion thereof as a probe, so as to obtain DNA that hybridizes with it. The term "a portion" of the DNA encoding the protein of the present invention is used to mean DNA having a length sufficient for the use as a probe, and specifically, such a length is 15 bp or longer, preferably 50 bp or longer, and more preferably 100 bp or longer.

Each of the aforementioned hybridization methods can be carried out in accordance with the method described in Molecular Cloning or the like.

The expression "DNA that hybridizes under stringent conditions" is used in the present specification to mean the nucleotide sequence of DNA obtained by the colony hybridization method, plaque hybridization method, Southern hybridization method, etc., using DNA used as a probe under stringent conditions. Examples of such stringent conditions may include conditions wherein a filter on which DNA derived from a colony or plaque or a fragment of the above DNA has been immobilized is subjected to hybridization in the presence of 0.7 to 1.0 M sodium chloride at 65°C by the colony hybridization method or plaque hybridization method, and thereafter, the filter is washed with a 0.1 to 2 x SSC solution (wherein 1 x SSC consists of 150 nM sodium chloride and 15 mM sodium citrate) at 65°C.

The thus isolated DNA encoding carbonyl reductase is inserted into a known expression vector such that it can be expressed therein, so as to obtain a carbonyl reductase expression vector. In addition, a transformant obtained by transformation with this expression vector is cultured, so as to obtain carbonyl reductase from the above transformant. Otherwise, such a transformant can also be obtained by incorporating DNA encoding carbonyl reductase into the chromosomal DNA of a known host such that the DNA can be expressed therein.

As a specific method for producing a transformant, the DNA of the present invention is introduced into a plasmid vector or a phage vector, which stably exists in microorganisms. Thereafter, the thus constructed expression vector is introduced into the microorganisms, or DNA encoding carbonyl reductase is directly introduced into a host genome, so as to transcribe and translate the gene information thereof.

At the time, if the DNA encoding carbonyl reductase does not contain a promoter capable of expressing in host microorganisms, it is necessary that a suitable promoter be incorporated into the side upstream of 5'-terminus of the DNA strand of the present invention, and that more preferably, a terminator be incorporated into the side downstream of 3'-terminus thereof. The types of such a promoter and a terminator are not particularly limited, as long as they are a promoter and a terminator that have been known to function in microorganisms used as hosts. A vector, a promoter, and a terminator that can be used in various types of microorganisms are described in detail in "*Biseibutsu*-*gaku Kiso Koza 8, Idenshi Kogaku* (Basic Course 8 in Microbiology, Genetic Engineering), Kyoritsu Shuppan Co., Ltd.," for example. In particular, with regard to yeasts, they are described in detail in Adv. Biochem. Eng. 43, 75-102 (1990), Yeast 8, 423-488 (1992), or the like, for example.

The types of host microorganisms that are targets of being transformed for expression of the carbonyl reductase of the present invention are not particularly limited, as long as the host itself does not affect the present reaction. Specific examples of such host microorganisms may include the following microorganisms:
bacteria whose host vector system has been established, which belong to genus Escherichia, genus Bacillus, genus Pseudomonas, genus Serratia, genus Brevibacterium, genus Corynebaterium, genus Streptococcus, genus Lactobacillus, etc.;
actinomycetes whose host vector system has been established, which belong to genus Rhodococcus, genus Streptomyces, etc.;
yeasts whose host vector system has been established, which belong to genus Saccharomyces, genus Kluyveromyces, genus Schizosaccharomyces, genus Zygosaccharomyces, genus Yarrowia, genus Trichosporon, genus Rhodosporidium, genus Hansenula, genus Pichia, genus Candida, etc.; and
molds whose host vector system has been established, which belong to genus Neurospora, genus Aspergillus, genus Cephalosporium, genus Trichoderma, etc.

Among the aforementioned microorganisms, preferred host microorganisms include genus Eschcrichia, genus Bacillus, genus Brevibacterium, and genus Corynebacterium. Particularly preferred microorganisms include genus Escherichia and genus Corynebacterium.

Procedures for production of a transformant, construction of a recombinant vector suitable for a host, and a method of culturing a host, can be carried out in accordance with techniques that are commonly used in the field of molecular biology, biological engineering, and genetic engineering (for example, the method described in Molecular Cloning).

Specific examples of preferred host microorganisms, preferred means for transformation of each type of microorganisms, a vector, a promoter, a terminator, and others will be given below. However, these examples are not intended to limit the scope of the present invention.

In the case of genus Escherichia, and in particular, *Eseherichia coli,* examples of a plasmid vector may include pBR and pUC plasmids. Examples of a promoter may include those derived from lac (β-galactosidase), trp (tryptophan operon), tac, trc (fusion of lac with tip), λ phage PL, and PR. Examples of a terminator may include those derived from trpA, phage, and rrnB ribosomal RNA.

In the case of genus Bacillus, examples of a vector may include a pUB110 plasmid and a pC194 plasmid. It is also possible to integrate it with a chromosome. Examples of a promoter and a terminator may include the promoters and terminators of enzyme genes, such as alkaline protease, neutral protease, or α-amylase.

In the case of genus Pseudomonas, examples of a vector may include a common host vector system established in *Pseudomonas putida, Pseudomonas cepacia*, or the like, a plasmid associated with decomposition of a toluene compound, and a euroxenous vector based on a TOL plasmid (including a gene necessary for autonomous replication derived from RSF1010) pKT240 (Gene, 26, 273-82 (1983)).

In the case of genus Brevibacterium, in particular, *Brevibacterium lactofermentum*, examples of a vector may include plasmid vectors such as pAJ43 (Gene, 39, 281 (1985)). Examples of a promoter and a terminator may include various types of promoters and terminators used in *Escherichia coli.*

In the case of genus Corynebacterium, in particular, *Corynebacterium glutamicum,* examples of a vector may include plasmid vectors such as pCS11 (Japanese Patent Application Laid-Open No. 57-183799) or pCB101 (Mol. Gen. Genet. 196, 175 (1984)).

In the case of genus Saccaromyces, in particular, *Saccharomyces cerevisiae*, examples of a vector may include YRp, YEp, YCp, and YIp plasmids. In addition, the promoters and terminators of various types of enzyme genes, such as alcohol dehydrogenase, glyceratdehyde-3-phosphate dehydrogenase, acid phosphatase, β-galactosidase, phosphoglycerate kinase, or enolase, are available.

In the case of genus Schizosaccharomyces, an example of a vector is a plasmid vector derived from *Shizosaccharomyces pombe* described in Mol. Cell. Biol. 6, 80 (1986). In particular, pAUR224 is commercially available from Takara Stitizo Co., Ltd., and thus, it can easily be used.

In the case of genus Aspergillus, *Aspergillus niger* and *Aspergillus oryzae* have vigorously been studied among molds. Integration thereof into a plasmid or chromosome is possible. Promoters derived from extracellular protease or amylase can be used (Trends in Biotechnology 7, 283-287 (1989)).

In addition, other than those as described above, host vector systems have been established depending on various types of microorganisms, and they can be used, as appropriate.

Moreover, other than microorganisms, various types of host-vector systems have been established in plants and animals. In particular, systems for allowing a large amount of heterologous protein to express in animals including insects such as silk worm (Nature 315, 592-594 (1985) or in plants such as rapeseeds, corns, or potato, and systems using a cell-free protein synthetic system such as an *Escherichia coli* cell-free extract or wheat germ, have been established, and these systems can preferably be used.

In the present invention, transformed cells having recombinant DNA obtained by incorporation of DNA having a nucleotide sequence encoding a protein having the amino acid sequence shown in SEQ ID NO: I into a vector which is obtained by the aforementioned method, or transformed cells obtained by incorporation of the above DNA into chromosomal DNA, or a product obtained by treating the above transformed cells and/or a culture solution thereof, are allowed to act on 2-pentanone or 2-hexanone acting as a reaction substrate, so as to asymmetrically reduce the carbonyl group of the above compound, thereby producing (S)-2-pentanol or (S)-2-hexanol.

In addition, in the present invention, a transformant having recombinant DNA obtained by incorporating into a vector, DNA having a nucleotide sequence encoding a protein, which has an amino acid sequence having homology of 50% or more with the amino acid sequence shown in SEQ ID NO: 1 and which has ability to reduce a carbonyl group to synthesize optically active alcohol, or transformed cells obtained by incorporation of the above DNA into chromosomal DNA, or a product obtained by treating the above transformed cells and/or a culture solution thereof, are allowed to act on 2-pentanone or 2-hexanone acting as a reaction substrate, so as to asymmetrically reduce the carbonyl group of the above compound, thereby producing (S)-2-pentanol or (S)-2-hexanol.

Moreover, in the present invention, microorganisms selected from the group consisting of genus Brettanomyces, genus Candida, genus Hortaea, genus Issatchenkia, genus Lodderomyces, genus Pichia, genus Rhodotorula, genus Arthrobacter, genus Brevibacterium, genus Crutobacterium, genus Geobacillus, genus Microbacterium, genus Ochrobactrum, genus Paracoccus, genus Rhizobium, and genus Rhodococcus, a product obtained by treating the above microorganisms, a culture solution of the above microorganisms, and/or a crude purified product or purified product of a carbonyl reductase fraction obtained from the above microorganisms, are allowed to act on 2-pentanone or 2-hexanone acting as a reaction substrate, so as to asymmetrically reduce the carbonyl group of the above compound, thereby producing (S)-2-pentanol or (S)-2-hexanol.

When such microorganisms are allowed to act on 2-pentanone to produce (S)-2-pentanol, microorganisms belonging to genus Brettanomyces, genus Candida, genus Hortaea, genus Lodderomyces, or genus Pichia, can preferably be used. Examples of microorganisms that are particularly preferably used herein may include *Brettanomyces bruxellensis*, *Candida tropicalis*, *Candida zeylanoides, Hortaea werneckii, Lodderomyces elongisporus, Pichia segobiefisis, Pichia spartinae, Arthrobacter globiformis, Arthrobacter oxydans*, *Arthrobacter polychromogenes*, *Curtobacterium flaccumfaciens*, *Geobacillus stearothermophilus*, *Microbacterium testaceum, Ochrobactrum anthropi,* Ochrobactrum sp. (*Pseudomonas ovalis*), and *Rhizobium radiobacter.* Specific examples of such microorganisms preferably used herein may include *Brettanomyces bruxellensis* NBRC 0629, *Brettanomyces bruxellensis* NBRC 0797, *Candida tropicalis* NBRC 0006, *Candida zeylanoides* CBS 6408, *Candida zeylanoides* JCM 1627, *Hortaea werneckii* NBRC 4875, *Lodderomyces elongisporus* NBRC 1676, *Pichia segobiensis* JCM 10740, *Pichia spartinae* JCM 10741, *Arthrobacter globiformis* NBRC 12137, *Arthrobacter oxydans* DSM 20120, *Arthrobacter polychromogenes* DSM 342, *Curtobacterium flaccumfaciens* ATCC 12813, *Geobacillus stearothermophilus* NBRC 12550, *Geobacillus stearothermophilus* IAM 11002, *Geobcillus stearothermophilus* IAM 11004, *Geobacillus stearothermophilus* IAM 12043, *Microbacterium testaceum* JCM 1353, *Ochrobuctrum anthropi* ATCC 49237, Ochrobactrum sp. (*Pseudomonas ovalis*) NBRC 12950, Ochrobactrum sp. (*Pseudomonas ovalis*) NBRC 12952, Ochrobactrum sp. (*Pseudomonas ovalis*) NBRC 12953, and *Rhizobium radiobacter* IAM 12048.

When such microorganisms are allowed to act on 2-hexanone to produce (S)-2-hexanol, microorganisms belonging to genus Brettanomyces, genus Candida, genus Issatchenkia, genus Lodderomyces, genus Pichia, or genus Rhodotorula, can preferably be used.

Of these, *Brettanomyces anomala, Candida famata, Candida krusei, Candida maltosa, Candida zeylanoides, Issatchenkia scutulata, Lodderomyces elongisporus, Pichia angusta, Pichia cactophila, Pichia segobiensis, Pichia trehalophila,* and *Rhodotorula minuta,* can particularly preferably be used.

Specific examples of microorganisms that are particularly preferably used herein may include *Brettanomyces anomala* NBRC 0627, *Candida famata* ATCC 10539, *Candida krusei* NBRC 1664, *Candida krusei* JCM 2284, *Candida krusei* JCM 2341, *Candida maltosa* NBRC 1977, *Candida zeylanoides* CBS 6408, Issatchenkia scutulata var. scutulata JCM 1828, *Lodderomyces elongisporus* NBRC 1676, *Pichia angusta* NBRC 1024, *Pichia angusta* NBRC 1071, *Pichia cactophila* JCM 1830, *Pichia segobiensis* JCM 10740, *Pichiatrehalophila* JCM 3651, *Rhodotorula minuta* NBRC 0879, Arthrobacter sp. DSM 20407, *Arthrobacter sulfurous (Brevibacterium sulfureum*) JCM 1338, *Brevibacterium butanicum* ATCC 21196, *Brevibacterium sulfureum* JCM 1485, *Curtobacterium flaccumfaciens* ATCC 12813, *Microbacterium keratanolyticum* NBRC 13309, *Microbacterium saperdae* JCM 1352, Microbacterium sp. NBRC 15615, *Ochrobactrum anthropi* ATCC 49237, Ochrobactrum sp. (*Pseudomonas ovalis*) NBRC 12952, Ochrobactrum sp. (*Pseudomonas ovalis*) NBRC 12953, *Paracoccus denitrificans* NBRC 12442, *Rhizobium radiobacter* IAM 12048, *Rhizobium radiobacter* IAM 13129, and Rhodococcus sp. ATCC 15960.

Moreover, 2-pentanone or 2-hexanone acting as a reaction substrate is used within the range of a substrate concentration generally between 0.01% and 90% w/v, and preferably between 0.1% and 30% w/v. Such a reaction substrate may be added all at once when the reaction is initiated. However, from the viewpoint of a reduction in the influence from substrate inhibition by enzyme or the improvement of the accumulation concentration of a product, it is desired to add a reaction substrate continuously or intermittently.

In the production method of the present invention, in order to allow the aforementioned transformed cells or microorganisms having carbonyl reduction activity to act on the carbonyl group-containing compound (reaction substrate) of 2-pentanone or 2-hexanone, the above transformed cells or microorganism cells may directly be used. However, products obtained by treating the above cells, such as a freeze dried product, a product obtained by physically or enzymatically disintegrating the above cells, a crude purified product or purified product obtained by extracting a carbonyl reductase fraction out of the above cells, or a product obtained by immobilizing the above on a carrier including polyacrylamide gel and carragheenan as representative examples, can also be used. With regard to the amount of transformed cells or microorganism cells, and/or a product obtained by treating the above cells, to be added to the reaction solution, the cells are added to the reaction solution such the concentration of the cells can be generally between approximately 0.1% and 50% w/v, and preferably between 1% and 20% w/v, at a wet cell mass weight. When a preparation such as an enzyme is used, the specific activity of the enzyme is obtained, and the preparation is added to the reaction such that the concentration of the cells can be within the above range.

In the production method of the present invention, it is preferable to add a coenzyme NADP⁺ or NADPH, or NAD⁺ or NADH. The concentration of such a coenzyme added is generally between 0.001 mM and 100 mM, and preferably between 0.01 and 10 mM.

When the aforementioned coenzyme is added, it is preferable in terms of the improvement of production efficiency that NADP⁺ (NAD⁺) generated from NADPH (NADH) be regenerated to NADPH (NADH). Examples of such a regeneration method may include: (1) a method utilizing the NADP⁺ (NAD⁺) reduction ability of host microorganisms themselves; (2) a method of adding in a reaction system, microorganisms having ability to generate NADPH (NADH) from NADH⁺ (NAD⁺) or a product obtained by treating the above microorganisms, or enzymes that can be used in regeneration of NADPH (NADH) (regenerating enzymes), such as glucose dehydrogenase, formate dehydrogenase, alcohol dehydrogenase, amino acid dehydrogenase, or organic acid dehydrogenase (malate dehydrogenase, etc.); and (3) a method of introducing the aforementioned regenerating enzyme genes that can be used in regeneration of NADPH (NADH) as well as the DNA of the present invention into a host, so as to allow them to express therein, when a transformant is produced.

Of these methods, it is preferable to add glucose, ethanol, formic acid, etc., to a reaction system in the method described in (1) above.

In addition, in the method described in (2) above, microorganism containing the aforementioned regenerating enzymes, cell mass-treated products such as a product obtained by treating a cell mass of the above microorganisms with acetone, a freeze dried product, or a physically or enzymatically disintegrated product, crude purified products of purified products obtained by extracting the above enzyme fraction, or products obtained by immobilizing products on a carrier including polyacrylamide gel or carragheenan as representative examples, may be used. Moreover, commercially available enzyme products may be used.

In this case, with regard to the specific amount of the above regenerating enzyme used, it is added at enzyme activity of generally 0.01 to 100 times, and preferably 0.5 to 20 times, when compared with the carbonyl reductase of the present invention.

Moreover, it is also necessary to add a compound acting as a substrate of the aforementioned regenerating enzyme, such as glucose in the case of using glucose dehydrogenase, formic acid in the case of using formate dehydrogenase, or ethanol or 2-propanol in the case of using alcohol dehydrogenase. The additive amount of such a compound is generally between 0.1 and 20 times molar equivalent, and preferably between 1 and 5 times molar equivalent, based on the amount of 2-pentanone or 2-hexanone used as a reaction material.

Furthermore, in the method described in (3) above, a of incorporating DNA encoding carbonyl reductase and the DNA of the aforementioned regenerating enzymes into the chromosome, a method of introducing both types of DNA into a single vector so as to transform a host with the vector, and a method of introducing both types of DNA into an each different vector and then transforming a host therewith, can be used. However, in the case of the method of introducing both types of DNA into an each different vector and then transforming a host therewith, it is necessary to select vectors while taking into consideration the incompatibility of both vectors.

When several genes are introduced into a single vector, it is also possible to apply a method of ligating regions associated with expression control, such as a promoter and a terminator, to each gene, or a method of allowing such genes to express as an operon containing several cistrons, such as lactose operon.

The production method of the present invention is carried out in an aqueous medium that contains a reaction substrate, transformed cells wherein DNA encoding carbonyl reductase has been allowed to express, a product obtained by treating the above cells, a culture solution of the above cells, and/or a crude purified product or purified product of a carbonyl reductase fraction obtained from the above cells, various types of coenzymes, which have been added as necessary, and a regeneration system thereof, or in the mixture consisting of the above-described aqueous medium and an organic solvent.

In addition, the production method of the present invention is carried out in an aqueous medium that contains a reaction substrate, microorganisms having carbonyl reductase activity belonging to genus Brettanomyces, genus Candida, genus Hortaea, genus Issatchenkia, genus Lodderomyces, genus Pichia, genus Rhodotorula, genus Arthrobacter, genus Brevibacterium, genus Crutobacterium, genus Geobacillus, genus Microbacterium, genus Ochrobactrum, genus Paracoccus, genus Rhizobium, or genus Rhodococcus, a product obtained by treating the above microorganisms, a culture solution of the above microorganisms, and/or a crude purified product or purified product of a carbonyl reductase fraction obtained from the above microorganisms, various types of coenzymes, which have been added as necessary, and a regeneration system thereof, or in the mixture consisting of the above-described aqueous medium and an organic solvent.

The aforementioned aqueous medium includes water and a buffer solution. Examples of a buffer solution may include sodium phosphate, potassium phosphate, tris, sodium acetate, and sodium citrate. Examples of an organic solvent used herein may include solvents in which a reaction substrate is highly dissolved, such as ethyl acetate, butyl acetate, toluene, chloroform, n-hexane, or dimethyl sulfoxide.

The of the present invention is carried out at a reaction temperature generally between 4°C and 60°C, and preferably between 10°C and 45°C, and at a pH generally between pH 3 and 11, and preferably between pH 5 and 8. The reaction time is generally between approximately 1 and 72 hours. In addition, the present method can be carried out using a membrane reactor or the like.

After completion of the reaction, optically active alcohol generated according to the method of the present invention can be purified by appropriately combining extraction with an organic solvent such as ethyl acetate or toluene, distillation, column chromatography, crystallization, or the like, after a cell mass or protein contained in the reaction solution has been separated by centrifugation, a membrane treatment, or the like.

### 2. Method for producing optically active 3-methyl carboxylic acid

In the production method of the present invention, optically active 3-methyl carboxylic acid represented by the following formula (5): 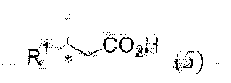 is produced by decarboxylation of optically active 1-methylalkyl malonic acid represented by the above formula (1) in the presence of a highly polar solvent and/or an additive for promoting decarboxylation.

In the above formula (1), R¹ represents a linear, branched, or cyclic alkyl group containing 3 to 5 carbon atoms, such as an n-propyl group, an n-butyl group, an n-pentyl group, an isopropyl group, an isobutyl group, an isoamyl group, or a cyclopentyl group. Of these, preferred alkyl groups include an n-propyl group, an n-butyl group, an n-pentyl group, an isopropyl group, and an isobutyl group. More preferred alkyl groups include an n-propyl group and an n-butyl group.

Further, * represents an asymmetric carbon, which may be either an R-form or an S-form. It is preferably an R-form, and the optical purity thereof is generally 80%ee or greater, and preferably 90%ee or greater. In particular, since high optical purity is required when it is used as a pharmaceutical material or intermediate, the optical purity thereof is further more preferably 95%ee or greater, and particularly preferably 99%ee or greater.

In the aforementioned decarboxylation reaction, the above compound may be heated in the presence of a highly polar solvent and/or an additive for promoting decarboxylation, or it may also be heated at a high temperature in the presence of a low polar solvent or in the absence of a solvent. In terms of the easiness of industrial application, it is preferable that the compound be heated in the presence of a highly polar solvent and/or an additive for promoting decarboxylation, while reducing a reaction temperature to be low. In general, such a decarboxylation reaction needs high temperature conditions between 150°C and 200°C. Such a high temperature is close to the service temperature limit of a common glass-lining reactor, and it also requires a long period of time for temperature rise and cooling. In addition, it requires a large amount of heat. Thus, a merit obtained from a low reaction temperature is considered to be extremely large. In addition, since optically active 1-methylalkyl malonic acid is generally a solid at ordinary temperature, when a reaction is carried out in no solvents, it is necessary that the compound be heated and melted after it has been added to a reactor. However, stirring cannot be carried out in a common reactor in a state where it contains only a solid, and if the compound is heated without being stirred, heat conductivity becomes extremely poor. Moreover, since there is a risk of reaction excursion due to partial temperature rise, the reaction performed in no solvents is not realistic. Thus, it is preferable to use a solvent so as to deal with optically active 1-methylalkyl malonic acid in the form of a solution or slurry.

On the other hand, since a large amount of carbon dioxide is generated as a result of the reaction, when such a reaction is carried out on an industrial scale, it is important to control the rate of generating carbon dioxide, so as to ensure safety.

From the viewpoint of the control of the rate of generating carbon dioxide, it is preferable that a solution in which optically active 1-methylalkyl masonic acid is dissolved, or optically active 1-methylalkyl malonic acid melted by heating, be continuously added dropwise, so as to carry out the reaction. In such dropping method, reaction control can easily be carried out, and a common reaction apparatus can be used. In addition, it is also possible to use optically active 3-methyl carboxylic acid generated as a result of the reaction as a solvent. In this case, since the reaction system does not contain a solvent or the like that should be eliminated after the reaction, it preferably results in low purification load.

Moreover, it is also possible to use a solvent having a boiling point higher than that of the generated optically active 3-methyl carboxylic acid. The use of a reaction solvent having a boiling point higher than that of the product enables prevention of the mixing of a lightly boiling component that causes a problem when a solvent with a light boiling point is used because optically active 3-methyl carboxylic acid is distilled as a first distillate component during distillation of the reaction, solution. Furthermore, by leaving a solvent with a high boiling point as a high boiling point diluent in a distillation container, a large amount of product of interest is distillated, thereby increasing distillation efficiency.

It is also possible to conduct reaction distillation, wherein an optically active 1-niethylalkyl masonic acid solution is added dropwise to a reactor under reduced pressure for reaction, and wherein the generated optically active 3-methyl carboxylic acid is continuously distilled. In the case of such a reaction distillation method, reaction control is easily carried out, and the heating time can be short. Thus, generation of impurity due to heating over a long period of time can preferably be suppressed.

It is also possible to convert optically active 1-methylalkyl masonic acid to Optically active 3-methyl carboxylic acid by a flow method comprising supplying an optically active 1-methylalkyl malonic acid solution into a reaction apparatus that has been heated. In the case of such a flow method, the supply of the solution to the reaction apparatus is controlled, so as to control the rate of generating carbon dioxide. In addition, since the heating time can be short, generation of impurity due to heating over a long period of time can preferably be suppressed. As a reaction apparatus used in a flow reaction, a tubular reactor, a thin-film distillatory, or a multistage tank flow reactor is preferably used. In the case of the thin-film distillatory, it is also possible to carry out the reaction under reduced pressure.

Examples of a solvent used in the aforementioned decarboxylation reaction may include: ether solvents such as butyl ether, tetrahydrouran, 1,2-dimethoxyethane, dioxane, polyethylene glycol, polyethylene glycol dimethyl ether, or polytetrabydrofuran; halogen solvents such as carbon tetrachloride or dichlorobenzene; alcohol solvents such as butanol or ethylene glycol; ester solvents such as ethyl acetate, dioctyl phthalate, diisononyl phthalate, ditridecyl phthalate, or trioctyl trimellitate; nitrile solvents such as acetonitrile or propionitrile; hydrocarbon solvents such as toluene, xylene, tetradecane, tridecane, liquid paraffin, monomethylnaphthalene, isopropylbiphenyl, dibenzyltoluene, hydrogenated triphenyl, or silicon oil; amide solvents such as dimethylformamide or N-methyl pyrrolidone; organic acid solvents such as formic acid or acetic acid; basic solvents such as pyridine, 2,6-lutidine, or triethylamine; dimethyl sulfoxide; and water. It may also be possible to mix several solvents selected from among these solvents at any given ratio. In order to allow a highly polar substrate or an additive to dissolve therein, highly polar solvents such as acetonitrile, tetrahydrofuran, dimethyl sulfoxide, pyridine, acetic acid, or water are preferable, and aprotic polar solvents having a great effect of accelerating the reaction, such as dimethyl sulfoxide or pyridine, are particularly preferable.

Moreover, high boiling point solvents such as tetradecane, tridecane, polyethylene glycol, polyethylene glycol dimethyl ether, polytetrahydrofuran, dioctyl phthalate, diisononyl phthalate, ditridecyl phthalate, trioctyl trimellitate, liquid paraffin, monomethylnaphthalene, isopropylbiphenyl, dibenxyltoluene, hydrogenated triphenyl, or silicon, oil, are used as high-boiling-point diluents during distillation.

Furthermore, as an amount of a solvent used, any given amount of solvent can be used. It is generally between 1 and 20 times, and preferably 1 and 5 times, at a volume based on the volume of a raw material substrate.

It is also possible to use an additive for reaction promotion as a solvent. In such a case, in order to dissolve or suspend optically active 1-methylalkyl malonic acid therein, the additive is generally used at a volume between 0.5 and 20 times based on the volume of a substrate. In order to reduce load such as the recover of a solvent via distillation, the amount of the additive is preferably between 0.5 and 3 times at a volume.

Examples of an additive used in the aforementioned decarboxylation reaction may include: mineral acids such as sulfuric acid or hydrochloric acid; inorganic salts such as sodium chloride or lithium chloride; organic salts such as sodium acetate or ammonium formate; cyanides such as sodium cyanide or copper cyanide; heavy metal salts such as copper chloride or iron chloride; heavy metal oxides such as copper oxide or silver oxide; organic bases such as pyridine, 2,6-lutidine, triethylamine, benzylamine, 1,8-diazabicyclo[5.4.0]-7-undecene, or 1,4-diazabicyclo[2.2.2]octane; inorganic bases such as sodium hydroxide, calcium hydroxide, or potassium carbonate; and acid anhydrides such as acetic anhydride or fumade anhydride. It may also be possible to mix these additives at any given ratio. Preferred examples of an additive may include a heavy metal salt, a heavy metal oxide, an organic base, an acid anhydride, and a mixture thereof, More preferred examples of an additive may include copper oxide, pyridine, 2,6-lutidine, acetic anhydride, and a mixture thereof, A particularly preferred example of an additive is pyridine, which is relatively inexpensive and has the effect of accelerating the reaction, and which can be separated from a product of interest by distillation.

The amount of an additive used is generally between 0.01% and 50% by weight based on the weight of a substrate. In order to avoid significant generation of carbon dioxide and reduce purification load, it is preferable that the amount of an additive used be controlled to the minimum necessary. Thus, the amount is preferably between 0.01% and 5% by weight.

The reaction temperature is generally between 30°C and 200°C. The necessary reaction temperature is different depending on reaction conditions such as the presence or absence of an additive or the type of an additive used. From the industrial viewpoint, a reaction performed at an extremely high temperature is restricted from the apparatus used, and thus, it becomes difficult to carry out the reaction. In addition, temperature rise and cooling require a long period of time. Hence, it is not desirable. The reaction temperature is preferably between 30°C and 150°C, and preferably between 30°C and 110°C.

Optimally active 3-methyl carboxylic acid obtained by the aforementioned reaction is preferably purified by methods such as distillation and/or extraction.

### 3. Method for active 1-methylalkyl malonic acid

The optically active 1-methyMkyl masonic acid of the present invention can be produced by converting optically active alcohol represented by the following formula (2): 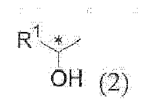 to a sulfonyloxy group, so as to obtain an optically active compound represented by the following formula (3): 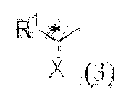 and then allowing the Optically active compound to react with a carbon nucleophile represented by the following formula (9): 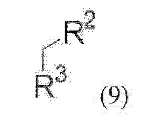 so as to obtain an optically active compound represented by the following formula (4): 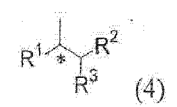 and then hydrolyzing the obtained optically active compound.

R¹ in the aforementioned formulas (2), (3), and (4), is the same as described above in the present specification.

In the aforementioned formulas (4) and (9), each of R² and R³ independently represents an ester group, a carboxyl group, or a cyano group, preferably represents an ester group. Herein, R² and R³ ma.y together form a cyclic structure such as 5-(1-methylalkyl)-2,2-dimethyl-1,3-dioxane-4,6-dione.

The alcohol component of the above ester group is not particularly limited, as long as it is a group that does not affect the reaction. Preferred examples may include: linear, branched, or cyclic alkyl alcohols such as methanol, ethanol, 1-butanoL 2-propanol, or cyclohexanol; and aryl alcohols such as phenol or naphthol. More preferred examples may include methanol ethanol.

In the above formula (3), X represents a sulfonyloxy group such as a methanesulfonyloxy group, p-toluenesulfonyloxy group, nitrobenzenesulfonyloxy group, chloromethanesulfonyloxy group, or trifluoromethanesulfonyloxy group. X preferably represents a methanesulfonyloxy group, p-toluenesulfonyloxy group, nitrobenzenesulfonyloxy group, chloromethanesulfonyloxy group, or trifluoromethanesulfonyloxy group. X more preferably represents a methanesulfonyloxy group or p-toluenesulfonyloxy group.

In the above formula (1) to (5), * represents an asymmetric carbon, and the optical purity thereof is generally 80%ee or greater, preferably 90%ee or greater, more preferably 95%ee, and particularly preferably 99%ee or greater. Absolute stereochemistry may be an R-form or S-form. It is preferably an S-form in the above formulas (2) and (3), and it is preferably an R-form in the above formulas (1), (4), and (5).

Optically active alcohol used as a raw material can arbitrarily be synthesized by a known method via an asymmetric reaction including the asymmetric reduction of a ketone corresponding thereto or resolution with lipase. However, such a resolution method has been problematic in that alcohol with an undesired stereochemistry or an ester thereof should be discarded. It is preferable that the above optically active alcohol be synthesized via the asymmetric reduction of a ketone wherein all the raw materials can be used. A more preferred synthetic method of synthesizing optically active alcohol comprises acting on a ketone, microorganism or transformed cells, wherein a protein having ability to reduce a carbonyl group even to an aliphatic ketone with a simple structure so as to synthesize Optically active alcohol at relatively high optical purity, or DNA encoding the above protein, has been expressed.

A method of converting optically active alcohol represented by the above formula (2) to a sulfonyloxy group is sulfonylation of a hydroxyl group.

Such sulfonylation of a hydroxyl group includes a method using: methanesulfonyl agents such as methanesulfonyl chloride or methanesulfonyl anhydride; p-toluenesulfonyl agents such as p-toluenesulfonyl chloride or p-toluenesulfonyl anhydride; trifluoromethanesulfonylation agents such as trifluoromethanesulfonic anhydride; and other agents.

Preferred sulfonyloxy groups include a methanesulfonyl group, a p-toluenesulfonyl group, a nitrobenzenesulfonyl group, a chloromethanesulfonyloxy group, and a trifluoromethanesulfonyl group. More preferred sulfonyloxy groups include a methanesulfonyl group and a p-toluenesulfonyl group, which are industrially inexpensively available.

The amount of a sulfonylation agent used in the aforementioned reaction is between I and 10 equivalents, and preferably between 1 and 2 equivalents, based on the amount of a substrate.

Examples of a solvent used herein may include: ether solvents such as ethyl ether, propyl ether, butyl methyl ether, or tetrahydrofuran; halogen solvents such as dichloromethane, chloroform, dichloroethatie, or chlorobenzene; ester solvents such as ethyl acetate or butyl acetate; hydrocarbon solvents such as hexane, benzene, or toluene; amide solvents such as dimethylformamide or N-methyl pyrrolidone; and nitrile solvents such as acetonitrile. It may also be possible that several solvents selected from among these solvents be mixed at any given ratio and be used. Preferred solvents include dichloromethane, ethyl acetate, and toluene, which are inexpensive and are easily recovered.

Any given amount of solvent may be used. The amount of a solvent is generally between 2 and 50 times volume, and preferably 3 and 10 times volume, based on the volume of a raw material substrate.

It is preferable to allow a base to coexist in the aforementioned reaction. Examples of a base used herein may include: organic bases such as triethylamine or pyridine; and inorganic bases such as sodium hydroxide, potassium carbonate, or sodium bicarbonate. Preferred examples are organic bases, and more preferred examples are triethylamine and pyridine.

The equivalent of a base used is an amount necessary to neutralize acid, which is generally generated as a by-product. It is between 1 and 10 equivalents, and preferably 1 and 2 equivalents, based on the amount of a substrate. A base may also be used as a solvent.

The reaction temperature is generally between -20°C and 100°C, and its optimum point is different depending on a leaving group to be introduced and/or reaction conditions. In the case of a particularly preferable methanesulfonyl or p-toluenesulfonyl group, the reaction temperature is preferably between 0°C and 40°C.

The reaction time can arbitrarily be determined. It is preferable to carry out the reaction within 10 hours from the viewpoint of suppression of a production cost.

Examples of a carbon nucleophile represented by formula (9) used in the above reaction may include a masonic ester, malonic acid, malononitrile, a malonic monoester, cyanoacetic acid, a cyanoacetic ester, and meldrum acid. Preferred examples may include a masonic ester, malononitrile, and a cyanoacetic ester. A more preferred example is a malonic ester, which is industrially inexpensive and is easily hydrolyzed.

The type of the alcohol component of the above ester is not particularly limited, as long as it is a group that does not affect the reaction. Preferred examples may include: linear, branched, or cyclic alkyl alcohols such as methanol, ethanol, 2-propanol, 1-butanol, or cyclohexanol; and aryl alcohols such as phenol or naphthol. More preferred examples may include methanol and ethanol.

The amount of a carbon nucleophile used is generally between 1 and 10 equivalents based on the amount of a substrate. In order to suppress generation of a dialkyl form generated as a result of the reaction of 2 molecules of substrate with a single carbon nucleophile, it is preferable to use a carbon nucleophile whose amount is much greater than that of the substrate. The amount of such a carbon nucleophile is between 1 and 3 equivalent, and preferably between 1.2 and 2.0 equivalents, based on the amount of a substrate.

Examples of a base used in the aforementioned reaction may include: metal hydride compounds such as sodium hydride or lithium hydride metal amide compounds such as lithium diisopropylamide or potassium hexamethyldisilazide: organic metal compounds such as n-butyllithium or isopropylmagnesium bromide; alkaline metals such as sodium, potassium, or lithium; alkaline earth metals such as calcium or magnesium; metal alkoxides such as sodium methoxide, sodium ethoxide, or potassium t-butoxide; and inorganic bases such as sodium hydroxide or potassium carbonate. Preferred examples may include metal hydride compounds, alkaline metals, and metal alkoxides. More preferred examples may include sodium hydride, sodium, and sodium methoxide.

The amount of a base used is between 1 and 10 equivalents, and preferably between 1 and 3 equivalents, based on the amount of a substrate.

Examples of a solvent used herein may include: ether solvents such as butyl methyl ether, tetrahydrofuran, 1.2-dimethoxyethane, or dioxane; halogen solvents such as dichloromethane, dichloroethane, or chlorobenzene; alcohol solvents such as methanol, ethanol, or 2-propanol; hydrocarbon solvents such as hexane or toluene; amide solvent such as dimethylformamide or N-methyl pyrrolidone; and dimethyl sulfoxide. It may also be possible that several solvents selected from among these solvents be mixed at any given ratio and be used. Preferred solvents include methanol, ethanol, dimethylformamide, tetrahydrofuran, and toluene. A more preferred solvent is tetrahydrofuran, which is a highly polar solvent, in which the reaction smoothly progresses, which is separable from an aqueous layer during extraction, and even the contamination thereof does not cause problems for the next step.

Any given amount of solvent may be used. The amount of a solvent is generally between 0.5 and 20 times volume based on the volume of a raw material substrate. However, as long as the reaction smoothy progresses, a smaller amount of solvent is preferably used to accelerate the reaction rate. It is between 1 and 8 times volume, and more preferably between 2 any 4 times volume, based on the volume of a substrate.

The reaction temperature is generally between 0°C and 100°C, and its optimum point is different depending on the type of a leaving group or carbon nucleophile or reaction conditions. In order to suppress racemization, the reaction is preferably carried out at a low temperature within a reaction time that is not too long. In the case of a reaction of the compound represented by the above formula (3) having a particularly preferable methanesulfonyl or p-toluenesulfonyl group with a masonic ester, the reaction temperature is preferably between 30°C 100°C, and more preferably between 50°C and 80°C.

The reaction largely on the type of a leaving group or carbon nucleophile or reaction conditions. It is preferable to carry out the reaction within 10 hours from the viewpoint of suppression of a production cost. However, since the optical purity of a product decreases if harsh reaction conditions such as a high temperature are applied to reduce the reaction time, it is necessary to select appropriate conditions such as the reaction time, temperature, or a solvent.

When the optically active compound represented by formula (4) obtained by the aforementioned reaction is converted to the optically active 1-methylalkyl malonic acid represented by the above formula (1), the above compound represented by formula (4) may be used in the form of a reaction solution without being purified. However, it is preferable to purify it by methods such as distillation and/or extraction, so as to obtain optically active 1-methylalkyl malonic acid having a higher purity.

An example of a method of converting the above compound to optically active 1-methylalkyl malonic acid is a method of converting an ester group and/or a cyano group to a carboxyl group by an acid treatment, an alkali treatment, or the like. It is possible to apply a stepwise method such as conversion of a cyano group to an ester group followed by hydrolysis. However, it is preferable to convert to optically active 1-methylalkyl malonic acid by a single step in a hydrated solvent because the number of steps can be reduced.

Examples of a reagent used in the aforementioned reaction may include: mineral acids such as sulfuric acid or hydrochloric acid; inorganic bases such as sodium hydroxide, potassium hydroxide, or potassium carbonate; and organic bases such as 1,8-diazabicyclo[5.4.0]undecane-7-en or sodium methoxide. Preferred examples of such a reagent may include sodium hydroxide, potassium hydroxide, sulfuric acid, and hydrochloric acid, which can industrially inexpensively be used.

Examples of a solvent used herein may include: ether solvents such as propyl ether, tetrahydrofuran, 1.,2-dimethyoxyethane, or dioxane; halogen solvents such as dichloromethane, dichloroethane, or chlorobenzene; alcohol solvents such as methanol ethanol, or ethylene glycol; hydrocarbon solvents such as hexane or toluene; amide solvents such as dimethylformamide or N-methyl pyrrolidone; organic acid solvents such as formic acid or acetic acid; dimethyl sulfoxide; and water. It may also be possible to mix several solvents selected from among these solvents at any given ratio and to use it, In order to carry out hydrolysis, it is preferable to use water, or a mixed solvent system consisting of water and a solvent capable of mixing with water. Examples of such a solvent capable of mixing with water may include tetrahydrofuran, methanol, ethanol, and acetic acid. A more preferred solvent is water.

Any given amount of solvent may be used. The amount of a solvent is generally between 1 and 20 times volume, and preferably 2 and 8 times volume, based on the volume of a raw material substrate.

The optically active 1-methylalkyl malonic acid represented by the above formula (1) is a compound having good crystallinity. When the optical purity thereof is not sufficient, it is preferable to increase the optical purity by crystallization. The optical purity after crystallization is preferably 90%ee or greater. Since a higher optical purity is required when the compound is used as a pharmaceutical material or intermediate, the optical purity is more preferably 95%ee or greater, and particularly preferably 99%ee or greater.

Such crystallization includes a common crystallization method of precipitating crystal from a solution such as an extract, recrystallization comprising dissolving by solvent addition, heating, or the like, crystals once precipitated by operations such as concentration, coaling, or solvent addition, and then precipitating crystal from the solution, and a method such as washing the generated crystals with a solvent.

Examples of a solvent used herein may include: ether solvents such as propyl ether, methyl butyl ether, tetrahydrofuran, 1,2-dimethoxyethane, or dioxane; halogen solvents such as dichloromethane, chloroform, dichloroethane, or chlorobenzene; alcohol solvents such as methanol, ethanol, 2-propanol, or ethylene glycol; ester solvents such as ethyl acetate or butyl acetate; nitrile solvents such as acetonitrile or propionitrile; hydrocarbon solvents such as hexane, heptane, benzene, toluene, or xylene; amide solvents as dimethylformamide or N-methyl pyrrolidone; dimethyl sulfoxide; and water. It may also be possible to mix several solvents selected from among these solvents at any given ratio and to use it. Preferred examples of a solvent may include propyl ether, hexane, heptane, benzene, toluene, and ethyl acetate, which are inexpensive and wherein the drying of crystal is easily carried out. More preferred examples may include heptane, toluene, xylene, and ethyl acetate, which have relatively high flash point and have good industrial handlability. A particularly preferred example is toluene, wherein the solubility of a product of interest is kept within an appropriate range, the solubility of impurities mixed is relatively high, and which enables precipitation of crystals by the single use thereof.

Any given amount of solvent may be used. The amount of a solvent is generally between 1 and 50 times volume based on the volume of a raw material substrate. Since the amount of a solvent is associated with the scale of crystallization or the cost of the solvent, it is appropriate to use an extremely small amount of solvent within a range in which the purpose for crystallization can be achieved. It is preferably 1 and 20 times volume, and more preferably between 1 and 10 times volume, based on the volume of a raw material substrate.

### 4. Optically active 1-methylalkyl malonic acid

The optically active 1-methylalkyl masonic of the present invention is a compound represented by the following formula (1):

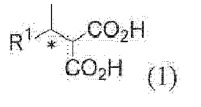

In the above formula (1), R¹ represents a linear, branched, or cyclic alkyl group containing 3 to 5 carbon atoms, such as an n-propyl group, an n-butyl group, an n-pentyl group, an isopropyl group, an isobutyl group, an isoamyl group, or a cyclopentyl group. Of these, R¹ preferably an n-propyl group, an n-butyl group, an n-pentyl group, an isopropyl group, and an isobutyl group. R¹ preferably an n-propyl group or an a-butyl group.

In addition, * represents an asymmetric carbon, Which may be either an R-form or S-form. It is preferably an R-form, and the optical purity thereof is generally 80%ee or greater, and preferably 90%ee or greater. Since high optical purity is required when the above compound is as a pharmaceutical or intermediate, the optical purity is more preferably 95%ee or greater, and particularly preferably 99%ee or greater.

The invention will be more specifically described in the following examples. However, the descriptions of these examples are not intended to limit the scope of the present invention.

### EXAMPLES

### Example A (Production of alcohol having optical activity, using microorganisms)

### (1) Isolation of microorganism generating (S)-2-pcntanol from 2-pentanone and microorganisms generating (S)-2-hexanol from 2-hexanone

Each of various types of cell strains shown in Table 1 was inoculated in 2.5 ml of liquid consisting of 5 g/L yeast extract (manufactured by Difco), 5 g/L POLYPPTONE(manufactured by Nihon Pharmaceutical Co., Ltd.), 3 g/L malt extract manufactured by Difco), and 20 g/L glucose (manufactured by Nihon Shokuhin Kako Co., Ltd.). It was then aerobically cultured at 30°C for 24 to 72 hours. 1 ml each of a culture solution was collected from each of the obtained culture solutions, and a cell mass was then collected by centrifugation. Thereafter, 0.04 ml of a Tris-HCl buffer solution (pH 7.0) and 0.028 ml of desalted water were added to the collected cell mass, so that the cell mass was sufficiently suspended therein. Thereafter, 0.05 ml of 100 g/L glucose and 0.02 ml of 12 g/L NADP⁺ (manufactured by Oriental Yeast Co., Ltd.) were added thereto. Subsequently, 0.01 ml of a solution obtained by dissolving 2-pentanone or 2-hexanone used as a reaction substrate in 2-propanol resulting in a concentration of 100 g/L, was further added thereto, and the obtained mixture was reacted at 30°C for 20 hours.

After completion of the reaction, the reaction solution was extracted with ethyl acetate. The generated (S)-2-pentanol or (S)-2-hexanol was then quantified. The product was quantified by gas chromatography (GC) using an ethyl acetate extract. Conditions for GC are as follows:
Column: β-DEX120 (manufactured by SUPELCO; 30 m x 0.25 mm ID, 0.25 µm film)
Carrier: He 1.5 ml/min, split 1/50
Column temperature: 50°C during .quantification of (S)-pentanol, and 65°C during quantification of (S)-hexanol
Temperature for injection: 250°C
Detection: FID 250°C
GC: ShimadzuGC-14A

The results of quantification of (S)-2-pentanol are shown in Table 1, and the results of quantification of (S)-2-hexanol are shown in Table 2.

**Table 1**

| | | | | |
|---|---|---|---|---|
| Yeast generating (S)-2-pentanol from 2-pentanone | | | | |

| Cell strain | | Product concentration (g/L) | Optical purity of product (% e.e.) | |
|---|---|---|---|---|
| *Brettanomyces bruxellensis* | NBRC 0629 | 1.07 | 100 | s |
| *Brettanomyces bruxellensis* | NBRC 0797 | 1.18 | 100 | s |
| *Candida trovicalis* | NBRC 0006 | 1.17 | 100 | s |
| *Candida zeylanoides* | CBS 6408 | 1.36 | 100 | s |
| *Candida zeylanoides* | JCM 1627 | 1.45 | 100 | s |
| *Hortaea werneckii* | NBRC 4875 | 1.34 | 100 | s |
| *Lodderomyces elongisporus* | NBRC 1676 | 1.25 | 100 | s |
| *Pichia segobiensis* | JCM 10740 | 1.16 | 100 | s |
| *Pichia spartinae* | JCM 10741 | 1.48 | 100 | s |
| Bacteria generating (S)-2-pc-ntanol from 2-pentanone | | | | |
| Cell strain | | Product. concentration, (g/L) | Optical purity of product (% e.e.) | |
| *Arthobacter globiformis* | NBRC 12137 | 1.36 | 99.1 | s |
| *Arthrobacter oxydans* | DSM 20120 | 1.25 | 100 | s |
| *Arthrobacter polychromogenes* | DSM 342 | 2.36 | 100 | s |
| *Curtobacterium flaccumfaciens* | ATCC 12813 | 1.94 | 99.7 | s |
| *Geobacillus stearothermophilus* | NBRC 12550 | 1.54 | 100 | s |
| *Geobacillus stearothermophlius* | IAM 11002 | 1.39 | 100 | s |
| *Geobacillus stearothermophlius* | IAM 11004 | 1.48 | 100 | s |
| *Geobacillus stearothermophilus* | IAM 12043 | 1.06 | 100 | s |
| *Microbacterium testaceum* | JCM 1353 | 1.2 | 100 | s |
| *Ochrobactrum anthropi* | ATCC 49237 | 1.47 | 100 | s |
| Ochrobactrum sp. | | | | |
| (*Pseudomonas ovalis*) | NBRC 12950 | 1.02 | 100 | s |
| Ochrobactrum sp. | | | | |
| (*Pseudomonas ovalis*) | NBRC 12952 | 1.49 | 100 | s |
| Ochrobactrum sp, | | | | |
| (*Pseudomonas ovalis*) | NBRC 12953 | 1.57 | 100 | s |
| *Rhizobium radiobacter* | IAM 12048 | 1.1 | 100 | s |

**Table 2**

| | | | | |
|---|---|---|---|---|
| Yeast generating (S)-2-hexanol from 2-hexanone. | | | | |

| Cell strain | | Product concentration (g/L) | Optical purity of product (%e.e.) | |
|---|---|---|---|---|
| *Brettatiomyces anomalus* | NBRC 0627 | 1.23 | 100 | s |
| *Candida famata* | ATCC 10539 | 1.20 | 93.0 | s |
| *Candida krusei* | JCM 2284 | 0.69 | 100 | s |
| *Candida krusei* | JGM 2341 | 0.88 | 100 | s |
| *Candida krusei* | NBRC 1664 | 1.10 | 100 | s |
| *Candida maltosa* | NBRC 1977 | 1.48 | 100 | s |
| *Candida zeylanoides* | CBS 6408 | 1.51 | 100 | s |
| *Issactchenkia scutulata var scutulata* | JCM 1828 | 1.07 | 91.8 | s |
| *Lodderomyces elongisporus* | NBRC1676 | 1.58 | 100 | s |
| *Pichia angusta* | NBRC 1024 | 1.16 | 100 | s |
| *Pichia angusta* | NBRC 1071 | 0.94 | 100 | s |
| *Pichia cactophlia* | JCM 1830 | 1.30 | 93.9 | s |
| *Pichia segobiensis* | JCM 10740 | 1.48 | 100 | s |
| *Pichia trehalophila* | JCM 3651 | 1.16 | 100 | s |
| *Rhodotorula minuta* | NBRC 0879 | 0.92 | 100 | s |
| Bacteria generating (S)-2-hexanol from 2-hexanone | | | | |

| Cell strain | | Product concentration (g/L) | Optical purity of product (% e.e.) | |
|---|---|---|---|---|
| Arthrobacter sp. | DSM 20407 | 1.04 | 100 | s |
| *Arthrobacter sulfureus* | | | | |
| | JCM 1338 | 1.26 | 100 | s |
| *(Brevibacterium sulfureum)* | | | | |
| *Brevibacterium butanicum* | ATCC 21196 | 1.12 | 100 | s |
| *Brevibacterium sulfureum* | JCM 1485 | 0.5 | 100 | s |
| *Curtobacterium flaccumfaciens* | ATCC 12813 | 1.29 | 100 | s |
| *Microbacterium keratanolyicum* | NBRC 13309 | 1.16 | 100 | s |
| *Microbacterium saperdae* | JCM 13S2 | 1.25 | 100 | s |
| Microbacterium sp. | NBRC 15615 | 0.68 | 100 | s |
| *Ochrobactrum anthropi* | ATCC 49237 | 0.91 | 100 | s |
| Ochrobactrum sp. (*Pseudomonas ovalis*) | NBRC 12952 | 1.58 | 100 | s |
| Ochrobactrum sp. (*Pseudomonas ovalis*) | NBRC 12953 | 1.73 | 100 | s |
| *Paracoccus denitrificans* | NBRC 12442 | 1.91 | 100 | s |
| *Rhizobium radiabacter* | 1AM 12048 | 0.91 | 100 | s |
| *Rhizobium radiobacter* | IAM 13129 | 0.79 | 99.3 | s |
| Rhodococcus sp. | | | | |
| | ATCC 15960 | 1.22 | 100 | s |
| *(Corynebacterium hydrocarboclastum)* | | | | |

### (2) Isolation of carbonyl reductase derived from Issatchankia scutulata var. scutulata J CM 1828 strain

The Issatchankia scutulata var. scutulata JCM1828 strain was cultured in 2 L of a medium (80 g of glucose, 20 g of yeast extract (manufactured by Difco), and 40 g/L peptone (manufactured by Kyokuto Pharmaceutical Industrial Co., Ltd.)), and cell mass was then prepared by centrifugation. 150 g of the obtained wet cell mass was suspended in 10 mM potassium phosphate buffer (pH 7) and 01. mM DTT (hereinafter simply referred to as a "buffer"), and it was then crushed with DYNO-MILL KDL (manufactured by Shimaru Enterprises Corp.). Thereafter, the cell mass residue was eliminated by centrifugation, so as to obtain a cell-free extract. Thereafter, PEG6000 was added to the cell-free extract to a concentration of 90 g/L, and the obtained mixture was then left at rest at 4°C for 1 hour. Thereafter, the precipitate was eliminated by centrifugation. The obtained supernatant was then subjected to anion exchange chromatography using DEAE Sepharose Fast Flow (manufactured by Amersham Biosciences), hydrophobic interaction chromatography using Butyl Sepharose 4 Fast Flow (manufactured by Amersham Biosciences), anion exchange chromatography using MonoQ (manufactured by Amersham Biosciences), and Gel filtration chromatography using Superdex 200 (manufactured by Amersham Biosciences), so that carbonyl reductase of interest could be purified to a single band in electrophoresis.

During purification, the activity of carbonyl reductase was measured by reacting the reaction solution containing an enzyme solution (100 mM Tris-HCl (pH 7.5), 0.32 mM NADPH, and 2 mM 1-acetoxy-3-chloro-2-propanone) at 37°C and then calculating the amount of NADPH consumed based on a decrease in the absorbance at 340 nm. For the measurement of the absorbance, SPECTRAmax 190 (manufactured by Molecular Devices) was used. It is to be noted that the activity necessary for consuming 1 nmol of NADPH for 1 minute in the aforementioned reaction was defined as 1 U.

The results regarding purification of carbonyl reductase derived from the Issatchankia scutulata var. scutulata JCM1828 strain are shown in Table 3.

**Table 3**

| Purification step | Total activity | Total protein amount | Specific activity | Purification | Yield |
|---|---|---|---|---|---|
| | (U) | (mg) | (U/mg) | magnification | (%) |
| Cell-free extract | 1360 | 16800 | 0.0810 | 1 | 100.0 |
| PEG6000 surfactant | 693 | 4680 | 0.148 | 1.83 | 51.0 |
| DEAE Sepharose FF | 857 | 266.0 | 3.22 | 39.8 | 63.0 |
| Butyl Sepharose 4 FF | 488 | 10.6 | 46.0 | 569 | 35.9 |
| MonoQ | 592 | 3.11 | 190 | 2351 | 43.5 |
| Superdex200 | 348 | 1.038 | 335 | 4141 | 25.6 |

The superdex200 active fraction at the purification step shown in the above Table 3 was analyzed by polyacrylamide gel electrophoresis (SDS-PAGE). As a result, it was found that the purified protein was almost a single band, and that the molecular weight thereof was approximately 40,000 Da.

### (3) Substrate specificity of IsADH1

With regard to various carbonyl compounds, a reaction solution (100 mM Tris-HCl (pH 7.5), 0.32 mM NADPH, and a 2 mM substrate) containing the carbonyl reductase solution purified in (2) above was prepared, and it was reacted at 37°C. The amount of NADPH consumed in the reaction solution was monitored based on the absorbance at 340 nm, so as to measure carbonyl reductase activity to each compound. For the measurement of the absorbance, SPECTRAmax 190 (manufactured by Molecular Devices) was used. The relative activity of carbonyl reductase to each substrate compound obtained when carbonyl reductase activity to 1-acetoxy-3-chloro-2-propanone was defined as 100 is shown in Table 4.

**Table 4**

| Substrate | Relative activity |
|---|---|
| 1-acetoxy-3-chloro-2-propanone | 100 |
| Ethyl 4-chloro-3-oxobutyrate | 212 |
| Ethyl 3-oxobutyrate | 25.5 |
| Acetoin | - |
| Diacetyl | - |
| 2,3-pentadione | Trace |
| Ethyl pyruvate | Trace |
| Ethyl 3-methyl-2-oxobutylate | Trace |
| Aectophenone | - |
| Propiophenone | Trace |
| Phenoxyacetone | - |
| 3-propionylpyridine | 11.2 |
| 2,2,2-trifluoroacetophenone | 53.8 |
| Phenylpyruvic acid | 12.2 |
| Ethyl benzoylacetate | Trace |
| Ethyl nicotinoylacetate | 20.7 |
| p-hydroxybenzaldehyde | - |
| p-chlorobenzaldehyde | 58.0 |
| 2-norbornanone | - |
| 3-quinuclidinone | Trace |

| | |
|---|---|
| -: Activity cannot be detected Trace: a slight level of activity is detested | |

### (4) Analysis of amino acid sequence of IsADH1

The fraction that contained carbonyl reductase of Superdex200 at the purification step shown in Table 3 obtained in (2) above was desalted and concentrated. Thereafter, the N-terminal amino acid thereof was analyzed by the Edman method, so as to determine the N-terminal amino acid sequence of 18 residues. The results are shown in SEQ ID NO: 4.

In addition, the purified carbonyl reductase was digested by the digestion method using lysyl endopeptidase *(*Tanpakushitsu Jikken Note (Protein Experiment Note), vol. 2, Yodosha Co., Ltd.), so as to obtain a peptide. The peptide was then separated using reverse phase HPLC (manufactured by Amersham Biosciences; µRPC C2/C18 PC3.2/3) for fractionation. The amino acid sequence of one type of the thus fractionated peptide peak was analyzed by the Edman method. The determined amino acid sequence is shown in SEQ ID NO: 5.

### (5) Analysis of sequence of DNA encoding IsADH1 and production of transformant

The Issatchankia scutulata var. scutulata JCM1828 strain was cultured in the medium described in (2) above, and a cell mass was then prepared.

Genomic DNA obtained from the cell mass was extracted with DNeasy tissue kit (manufactured by Qiagen) and was then purified. Based on the obtained genomic DNA, cDNA was synthesized using reverse transcriptase, Superscript II Reverse Transcriptase (manufactured by Invitrogen), in accordance with the protocols included therewith.

Two types of primers were synthesized. That is, a sense degenerate primer was synthesized based on the N-terminal amino acid sequence shown in SEQ ID NO: 4 obtained in (4) above, and an antisense degenerate primer was synthesized based on the internal amino acid sequence shown in SEQ ID NO: 5. The nucleotide sequences thereof are shown in SEQ ID NOS: 6 and 7, respectively. Using such two types of primers, degenerate PCR was performed on the cDNA of the Issatchankia scutulata var. scutulata JCM1828 strain. As a result, an amplified fragment of approximately 350 bp was observed.

This DNA fragment was subjected to agarose gel electrophoresis, so as to cut out a band of approximately 350 bp. It was then purified using MinElute Gel Extraction Kit (manufactured by Qiagen) and recovered. The obtained DNA fragment was ligated to the pGEM-Teasy Vector (manufactured by Promega), and the *Escherichia coli* DH5α strain (manufactured by Toyobo) was then transformed therewith. The transformant strain was allowed to grow on an LB agar medium containing ampicillin (100 µg/ml). Thereafter, using several colonies, colony direct PCR was carried out using the T7 primer (manufactured by Promega) and the SP6 primer (manufactured by Promega), and the size of the inserted fragment was then confirmed. A colony, into which a DNA fragment of interest was considered to be inserted, was cultured in an LB medium containing 100 µg/ml ampicillin. Thereafter, a plasmid was purified using QIAPrep Spin Mini Prep Kit (manufactured by Qiagen).

Using the purified plasmid, the nucleotide sequence of the inserted DNA was analyzed by the dye terminator method. The determined nucleotide sequence is shown in SEQ ID NO: 8.

Subsequently, based on the genomic DNA of the Issatchankia scutulata var. scutulata JCM1828 strain, cDNA used in RACE reaction was synthesized according to the method described in Molecular Cloning, and thereafter, 5'- and 3'-RACE reactions were carried out by the method described in the same above publication. For the reactions, two types of gene specific primers shown in SEQ ID NOS: 9 and 10 which were designed based on the aforementioned nucleotide sequence were used.

As a result of the sequence analysis of amplified gene fragments via the RACE reactions, the putative CDNA sequence of the present carbonyl reductase is shown in SEQ ID NO: 11, and an amino acid sequence encoded by the above DNA is shown in SEQ ID NO: 1. A nucleotide sequence encoding the amino acid sequence shown in SEQ ID NO: 1 is shown in SEQ ID NO: 2.

Subsequently, based on the above sequence shown in SEQ ID NO: 11, the nucleotide sequence shown in SEQ ID NO: 12 and the nucleotide sequence shown in SEQ ID NO: 13 were synthesized as primers used in cloning. Using 100 µl of a reaction solution that contained 50 pmol each of the aforementioned primers, 1000 nmol each of DNTP, 250 ng of the cDNA of the Issatchankia scutulata var. scutulata JCM1828 strain, 10 µl of ExTaq DNA polymerase 10 x buffer solution (manufactured by Takara Bio), and 5 units of ExTaq DNA polymerase (manufactured by Takara Bio), a cycle consisting of denaturation (95°C, 1 minute), annealing (58°C, 1 minute), and elongation (72°C, 1 minute) was repeated 30 times, employing PTC-200 (manufactured by MJ Research). A portion of the PCR reaction solution was analyzed by agarose gel electrophoresis. As a result, a specific band was detected.

The aforementioned reaction solution was purified using MinElute PCR Purification kit (manufactured by Qiagen). The purified DNA fragment was digested with restriction enzymes EcoRI and XbaI, and the resultant was then subjected to agarose gel electrophoresis, and a band portion of interest was cut out. The band portion was purified using Qiagen Gel Extraction kit (manufactured by Qiagen), and was then recovered. The obtained DNA fragment was ligated with pUC118 that had been digested with EcoRI and XbaI, using Ligation high (manufactured by Toyobo), and the *Escherichia coli* JM109 strain was then transformed with the ligate.

The transformant strain was allowed to grow on an LB agar medium containing 50 µg/ml ampicillin, colony direct PCR was then carried out, so as to confirm the size of the inserted fragment.

A transformant, into which a DNA fragment of interest was considered to be inserted, was cultured in an LB medium containing 50 µg/ml ampicillin. Thereafter, a plasmid was purified using QIAPrep Spin Mini Prep Kit (manufactured by Qiagen), so as to obtain pUCIsADH1.

The nucleotide sequence of the DNA inserted into the plasmid was analyzed by the dye terminator method. As a result, it was found that the nucleotide sequence of the inserted DNA fragment corresponded to the nucleotide sequence shown in SEQ ID NO: 2.

### (6) Synthesis of (S)-2-pentanol, using Escherichia coli transformed with DNA encoding IsADH1

The transformant obtained in (5) above was cultured in 10 series at 30°C for 30 hours in 100 ml of Circle Grow medium (manufactured by BIO 101) containing ampicillin (50 µg/ml). The obtained cell was collected by centrifugation, and (S)-2-pentanol was synthesized by the following method using 2-pentanone as a substrate.

20 mg of 0.6 g/L NADP⁺ (manufactured by Oriental Yeast Co., Ltd.), 10 ml of 1M Tris-HCl buffer (pH 7.0), 40 ml of 100 g/L glucose, 20 mg of glucose dehydrogenase (manufactured by Amano Seiyaku; 76 units/mg), and 1 g of 2-pentanone (manufactured by Tokyo Chemical Industry Co., Ltd.; Neat) were added to 10 g of the aforementioned cell mass. The obtained mixture was reacted at 30°C for 8 hours. The pH during the reaction was kept at pH 7.0 by addition of 2 M sodium carbonate. After completion of the reaction, the reaction solution was extracted with ethyl acetate, and the generated (S)-2-pentanol was then quantified.

Quantification was carried out using the ethyl acetate solution by gas chromatography (GC).

Conditions for GC are as follows:
Column: β-DEX120 (manufactured by SUPELCO; 30 m x 0.25 mm ID, 0.25 µm film)
Carrier: He 1.5 ml/min, split 1/50
Column temperature: 50°C
Temperature for injection: 250°C
Detection: FID 250°C
GC: Shimadzu GC-14A

As a result of the measurement, it was found that the yield of (S)-2-pentanol was 0.99 g, and that the optical purity thereof was greater than 99.0%e.e.

### (7) Synthesis of (S)-2-hexanol, using Escherichia coli transformed with DNA encoding ISADH1

Using the transformant obtained in (6) above, (S)-2-hexanol was synthesized from 2-hexanone acting as a substrate by the following method.

20 mg of 0.6 g/L NADP⁺ (manufactured by Oriental Yeast Co., Ltd.), 10 ml of 1M Tris-HCl buffer (pH 7.0), 40 ml of 100 g/L glucose, 20 mg of glucose dehydrogenase (manufactured by Amano Seiyaku; 76 units/mg), and 1 g of 2-hexanone (manufactured by Tokyo Chemical Industry Co., Ltd.; Neat) were added to 10 g of the aforementioned cell mass. The obtained mixture was reacted at 30°C for 6 hours. The pH during the reaction was kept at pH 7.0 by addition of 2 M sodium carbonate. After completion of the reaction, the reaction solution was extracted with ethyl acetate, and the generated (S)-2-hexanol was then quantified.

Quantification was carried out using the ethyl acetate solution by gas chromatography (GC).

Conditions for GC are as follows:
Column: β-DEX120 (manufactured by SUPELCO; 30 m x 0.25 mm ID, 0.25 µm film)
Carrier: He 1.5 ml/min, split 1/150
Column temperature: 65°C
Temperature for injection: 250°C
Detection: FID 250°C
GC: Shimadzu GC-14A

As a result of the measurement, it was found that the yield of (S)-2-hexanol was 0.99 g, and that the optical purity thereof was greater than 99.0%e.e.

### (8) Synthesis of (S)-2-pentanol, using Escherichia coli transformed with DNA encoding IsADH1 (scale-up)

Using the transformant obtained in (6) above, (S)-2-pentanol was synthesized from 2-pentanone acting as a substrate by the following method.

84 mg of NADP⁺ (manufactured by Oriental Yeast Co., Ltd.), 140 ml of 1M Tris-HCl buffer (pH 7.0), 118 g of glucose, 40 mg of glucose dehydrogenase (manufactured by Amano Seiyaku; 76 units/mg), and 28 g of 2-pentanone (manufactured by Tokyo Chemical Industry Co., Ltd.; Neat) were added to 140 g of the aforementioned cell mass (corresponding to approximately 42 g of dry cell mass weight). Thereafter, desalted water was added to the mixture to a reaction volume of 1.4 L. The obtained mixture was reacted at 30°C for 16 hours. The pH during the reaction was kept at pH 7.0 by addition of 2 M sodium carbonate. After completion of the reaction, the reaction solution was extracted with ethyl acetate, and the generated (S)-2-pentanol was then quantified. As a result of the measurement, it was found that the yield of (S)-2-pentanol was 17.2 g, and that the optical purity thereof was greater than 99.0%e.e.

### (9) Synthesis of (S)-2-hexanol, using Escherichia coli transformed with DNA encoding IsADH1 (scale-up)

Using the transformant obtained in (6) above, (S)-2-hexanol was synthesized from 2-hexanone acting as a substrate by the following method.

30 mg of NADP⁺ (manufactured by Oriental Yeast Co., Ltd.), 50 ml of 1M Tris-HCl buffer (pH 7.0), 54 g of glucose, 20 mg of glucose dehydrogenase manufactured by Amano Seiyaku; 76 units/mg), and 15 g of 2-hexanone (manufactured by Tokyo Chemical Industry Co., Ltd.; Neat) were added to 50 g of the aforementioned cell mass (corresponding to approximately 15 g of dry cell mass weight). Thereafter, desalted water was added to the mixture to a reaction volume of 500 ml. The obtained mixture was reacted at 30°C for 7.5 hours. The pH during the reaction was kept at pH 7.0 by addition of 2 M sodium carbonate. After completion of the reaction, the reaction solution was extracted with ethyl acetate, and the generated (S)-2-hexanol was then quantified. As a result of the measurement, it was found that the yield of (S)-2-hexanol was 15.1 g, and that the optical purity thereof was greater than 99.0%e.e.

### Example B (Production of optically active carboxylic acid by chemical synthesis) (Example 1) Synthesis of (S)-2-methanesulfonyloxy pentane

4.14 g (47.0 mmol, 99.1%ee) of (S)-2-pentanol, 9.8 ml (71 mmol) of triethylamine, and 41 ml of dichloromethane were added to a 200-ml three-neck flask. The mixed solution was cooled on ice, and 4.36 ml (56.4 mmol) of methanesulfonyl chloride was then added dropwise thereto. Thereafter, the obtained mixture was stirred for 30 minutes. Thereafter, 40 ml of a saturated ammonium chloride aqueous solution and 20 ml of water were added to the reaction solution, and the reaction was terminated. The mixture was extracted with 80 ml of diethyl ether. An organic layer was then washed with 20 ml of a saturated ammonium chloride aqueous solution and 20 ml of brine, and was then dried with magnesium sulfate. The solvent was distilled away, so as to obtain 8.5 g of crude (S)-2-methanesulfonyloxy pentane. The obtained compound was used in the subsequent reaction without being purified.
¹H-NMR(400MHz,CDCl₃) δ0.95(t,J=7.2Hz, 3H), 1.42(d,J=6.3Hz,3H), 1.34-1.50(m,2H), 1.50-1.63(m,1H), 1.67-1.77(m,1H), 3.00(s,3H), 4.77-4.86(m,1H).

### (Example 2) Synthesis of (R)-diethyl(1-methylbutyl)malonate

8.5 g of the crude (S)-2-methanesulfonyloxy pentane obtained in Example 1, 14.2 g (88 mmol) of diethyl malonate, and 22 ml of DMF were added to a 200-ml eggplant-shaped flask. The mixed solution was cooled on ice bath, and 3.5 g (88 mmol) of 60% sodium hydride in mineral was then added thereto. Thereafter, the obtained mixture was stirred at 60°C for 5 hours, and then at 80°C for 3 hours. Thereafter, 40 ml of a saturated ammonium chloride aqueous solution and 10 ml of water were added to the reaction solution at room temperature, and the reaction was terminated. The mixture was extracted with 100 ml of ethyl acetate. An organic layer was then washed with 20 ml of a saturated ammonium chloride aqueous solution, 20 ml of water, and 20 ml of brine, and was then dried with magnesium sulfate. The solvent was distilled away, and the residue was then purified by silica gel column chromatography, so as to obtain 7.77 g (colorless oil; 33.8 mmol; yield: 72%) of (R)-diethyl(1-methylbutyl)malonate.
¹H-NMR(400MHz,CDCl₃) δ0.89(t,J=6.9Hz,3H), 0.98(d,J=6.6Hz,3H), 1.27(t,J=7.1Hz,6H), 1.15-1.46(m,4H), 2.20-2.31(m,1H), 3.22(d.J=8.1Hz,1H), 4.19(q,J=7.1Hz,4H).

### (Example 3) Synthesis of (R)-(1-methylbutyl)malonic acid

7.77 g (33.5 mmol) of the (R)-diethyl(1-methylbutyl)malonate obtained in Example 2, 16.1 g (100 mmol) of a 25% sodium hydroxide aqueous solution, 3.9 ml of ethanol, and 15.4 ml of water were added to a 200-ml eggplant-shaped flask. The mixed solution was reacted at 60°C for 1.5 hours, and 9 ml of concentrated hydrochloric acid was added to the reaction solution at room temperature, so as to convert the solution to be acidic. Sodium chloride was added to the mixed solution for saturation, and it was then extracted with 100 ml of ethyl acetate. An aqueous layer was extracted again with 50 ml of ethyl acetate, and an organic layer was dried with sodium sulfate. The solvent was distilled away, and the residue was then crystallized from 58 ml of hexane and 5.8 ml of ethyl acetate, so as to obtain 4.73 g (white platy crystal; 2.71 mmol; yield: 81%) of (R)-(1-methylbutyl)malonic acid. As a result of chiral analysis, it was found that the optical purity thereof was 99.3%ee (Supelco β-DEX120, inj. 300°C, FID 250°C, Det. 250°C, Oven 130°C; 3-methyl hexanic acid generated by decarboxylation during injection was analyzed).
¹H-NMR(400MHz,CDCl₃) δ0.91(t,J=6.8Hz,3H), 1.06(d,J=6.8Hz,3H), 1.22-1.40(m,2H). 1.40-1.58(m,2H), 2.25-2.37(m,1H), 3.37(d,J=7.6Hz,1H), 10.67(brs,2H).

### (Example 4) Synthesis of (S)-2-methanesulfonyloxy hexane

6.3 g (62 mmol, 99.7%ee) of (S)-2-hexanol, 13 ml (93 mmol) of triethylamine, any 126 ml of ethyl acetate were added to a 300-ml three-neck flask. The mixed solution was cooled on ice bath, and 5.7 ml (74 mmol) of methanesulfonyl chloride was then added dropwise thereto. Thereafter, the obtained mixture was stirred for 30 minutes. Thereafter, 40 ml of a saturated ammonium chloride aqueous solution and 20 ml of water were added to the reaction solution, and the reaction was terminated. An aqueous layer was separated. An organic layer was then washed with 20 ml of a saturated ammonium chloride aqueous solution and 20 ml of brine, and was then dried with magnesium sulfate. The solvent was distilled away, so as to obtain 10.8 g of crude (S)-2-methanesulfonyloxy hexane. The obtained compound was used in the subsequent reaction without being purified.
¹H-NMR(400MHz,CDCl₃) δ0.92(t,J=7.1Hz,3H), 1.25-1.45(m,4H), 1.42(d,J=6.3Hz,3H), 1.55-1.65(m,1H), 1.68-1.79(m,1H), 3.00(s,3H), 4.75-4.84(m,1H).

### (Example 5) Synthesis of (R)-diethyl (R)-(1-methylpentyl)malonate

10.8 g of the crude (S)-2-methanesulfonyloxy hexane obtained in Example 4, 19.2 g (120 mmol) of diethyl malonate, and 32 ml of DMF were added to a 200-ml eggplant-shaped flask. The mixed solution was cooled on ice bath, and 4.8 g (120 mmol) of 60% sodium hydride in mineral was then added thereto. Thereafter, the obtained mixture was stirred at 60°C for 1 hour, and then at 80°C for 3 hours. Thereafter, 50 ml of a saturated ammonium chloride aqueous solution and 10 ml of water were added to the reaction solution at room temperature, and the reaction was terminated. The mixture was extracted with 100 ml of ethyl acetate. An organic layer was then washed with 30 ml of water twice, and with 30 ml of brine, and was then dried with magnesium sulfate. The solvent was distilled away, and the residue was then purified by silica gel column chromatography, so as to obtain 12.0 g (colorless oil; 49 mmol; yield: 82%) of (R)-diethyl(1-methylpentyl)maonate.
¹H-NMR(400MHz,CDCl₃) δ0.89(t,J=6.9Hz,3H), 0.98(d,J-6.6Hz,3H), 1.27(t,J=7.1Hz,6H), 1.15-1.45(m,6H), 2,17-2.29(m,1H), 3.22(d,J=8.1H.z,1H), 4.19(q,J=7.1Hz,4H).

### (Example 6) Synthesis of (R)-(1-methylpentyl)malonic acid

11.7 g (48 mmol) of (R)-diethyl(1-methylpentyl)malonate obtained in Example 5, 23 g (144 mmol) of a 25% sodium hydroxide aqueous solution, 5.9 ml of ethanol, and 24 ml of water were added to a 100-ml eggplant-shaped flask. The mixed solution was reacted at 60°C for 2 hours, and 13 ml of concentrated hydrochloric acid was added to the reaction solution at room temperature, so as to convert the solution to be acidic. Sodium chloride was added to the mixed solution for saturation, and it was then extracted with 120ml of ethyl acetate. An aqueous layer was extracted again with 40 ml of ethyl acetate, and an organic layer was dried with sodium sulfate. The solvent was distilled away, and the residue was then crystallized from 90 ml of hexane and 18 ml of ethyl acetate, so as to obtain 6.5 g (white columnar crystal; 35 mmol; yield: 72%) of (R)-(1-methylpentyl)malonic acid.
¹H-NMR(400MHz,CDCl₃) δ0.90(t,J=6.7Hz,3H), 1.07(d,J=6.8Hz,3H), 1.21-1.41(m,5H), 1.46-1.56(m,1H), 2.22-2.33(m,1H), 3.39(d,J-7.3Hz,1H).

### (Example 7) Synthesis of (R)-3-methylhexanoic acid

4.56 g (26.2 mmol) of (R)-(1-methylbutyl)malonic acid obtained in Example 3 was added to a 50-ml eggplant-shaped flask, and the temperature was then increased to 180°C. After generation of gas had been terminated, the mixture was further stirred for 10 minutes, and it was then cooled to room temperature. The obtained crude product was subjected to vacuum distillation, so as to obtain 2.05 g (colorless oil; boiling point: 77°C/3 mmHg; 15.7 mmol; yield: 60%) of (R)-3-methylhexanoic acid, As a result of chiral analysis, it was found that the optical purity thereof was 99.2%ee (SUPELCO β-DEX120, inj. 250°C, FID 250°C, Det. 250°C, Oven 130°C).
¹H-NMR(400MHz,CDCl₃) δ0.90(t,J=7.1Hz,3H), 0.96(d,J=6.6Hz,3H), 1.16-1.44(m,4H), 1.91-2.05(m,1H), 2.15(dd,J=14.9,8.1Hz,1H), 2.35(dd,J=14.9,5.8Hz,1H), 11.00(brs,1H).

### (Example 8) Synthesis of (R)-3-methylheptanoic acid

3.00 g (15.9 mmol) of (R)-(1-methylpentyl)malonic acid obtained in Example 6 was added to a 20-ml eggplant-shaped flask, and the temperature was then increased to 180°C. After generation of gas had been terminated, the mixture was further stirred for 10 minutes, and it was then cooled to room temperature. The obtained crude product was subjected subjected to vacuum distillation, so as to obtain 1.69 g (colorless oil; boiling point: 86°C/4 mmHg; 11.7 mmol; yield: 74%) of (R)-3-methylheptanoic acid.
¹H-NMR(400MHz,CDCl₃) δ0.89(t,J=6.8Hz,3H), 0.97(d,J=6.8Hz,3H), 1.17-1,39(m.6H), 1.88-2.03(m,1H), 2.14(dd,J=14.9,8.1Hz,1H), 2.35(dd,J=14.9,5.9Hz,1H), 11.36(brs,1H).

### (Example 9) Synthesis of (R)-3-methylhexanoic acid (pyridine solvent)

1.00 g (5.8 mmol) of (R)-(1-methylbutyl)malomc acid and 2 ml of pyridine were added to a 15-ml stoppered test tube under nitrogen atmosphere, and the temperature was then increased. The reaction was initiated at 90°C, and the temperature was then increased to 130°C over 115 minutes. Thereafter, the reaction solution was further stirred for 30 minutes, and it was then cooled to room temperature. As a result of the analysis by HPLC, the conversion rate was 100.0%.

### (Example 10) Synthesis of (R)-3-methylhexanoic acid (DMSO solvent)

2.00 g (11.5 mmol) of (R)-(1-methylbutyl)malonic acid and 4 ml of DMSO were added to a 15-ml stoppered test tube under nitrogen atmosphere, and the temperature was then increased. The reaction was initiated at 100°C, and the temperature was then increased to 140°C over 75 minutes. Thereafter, the reaction solution was further stirred for 20 minutes, and it was then cooled to room temperature. As a result of the analysis by HPLC, the conversion rate was 100.0%,

### (Example 11) Synthesis of (R)-3-methylhexanoic acid (addition of pyridine)

2.00 g (11.5 mmol) of (R)-(1-methylbutyl)malonic acid and 182 mg (2.3 mmol) of pyridine were added to a 15-ml stoppered test tube under nitrogen atmosphere, and the temperature was then increased. The reaction was initiated at 100°C, and the temperature was then increased to 150°C over 95 minutes. Thereafter, the reaction solution was further stirred for 15 minutes, and it was then cooled to room temperature. As a result of the analysis by HPLC, the conversion rate was 100.0%.

### (Example 12) Synthesis of (R)-3-methylhexanoic acid (addition of DABCO)

2.00 g (11.5 mmol) of (R)-(1-methylbutyl)matomc acid and 258 mg (2.3 mmol) of 1,4-diazabicyclo[2.2.2]octane (DABCO) were added to a 15-ml stoppered test tube under nitrogen atmosphere, and the temperature was then increased. The reaction was initiated at 100°C, and the temperature was then increased to 140°C over 95 minutes. Thereafter, the reaction solution was further stirred for 15 minutes, and it was then cooled to room temperature. As a result of the analysis by HPLC, the conversion rate was 100.0%.

### (Example 13) Synthesis of (R)-3-methylhexanoic acid (addition of DBU)

2.00 g (11.5 mmol) of (R)-(1-methylbutyl)malomc acid and 350 mg (2.3 mmol) of 1,8-diazabicyclo[5.4.0]-7-undecene (DBU) were added to a 15-ml stoppered test tube under nitrogen atmosphere, and the temperature was then increased. The reaction was initiated at 110°C, and the temperature was then increased to 140°C over 50 minutes. Thereafter, the reaction solution was further stirred for 20 minutes, and it was then cooled to room temperature. As a result of the analysis by HPLC, the conversion rate was 100.0%.

### (Example 14) Synthesis of (R)-3-methylhexanoic acid (acetic anhydride catalyst/pyridine solvent)

1.00 g (5.8 mmol) of (R)-(1-methylbutyl)malonic acid and 2 ml of pyridine were added to a 15-ml stoppered test tube under nitrogen atmosphere, and 294 mg (2.88 mmol) of acetic anhydride was then added thereto. The mixture was stirred at room temperature for 2 hours and then at 40°C for 1 hour, and it was then cooled to room temperature. As a result of the analysis by HPLC, the conversion rate was 99.8%.

### (Example 15) Synthesis of (R)-3-methylhexanoic acid (addiction of sulfuric acid)

2.00 g (11.5 mmol) of (R)-(1-methylbutyl)malonic acid and 113 mg (1.15 mmol) of sulfuric acid were added to a 15-ml stoppered test tube under nitrogen atmosphere, and the temperature was then increased. The reaction was initiated at 130°C, and the temperature was then increased to 180°C over 100 minutes. Thereafter, the reaction solution was further stirred for 20 minutes, and it was then cooled to room temperature. As a result of the analysis by HPLC, the conversion rate was 100.0%.

### (Example 16) Synthesis of (R)-3-methylhexanoic acid (addition of Fe₃O₄)

2.00 g (11.5 mmol) of (R)-(1-methylbutyl)malonic acid and 40 mg (2 wt%) of iron oxide (Fe₃O₄) were added to a 15-ml stoppered test tube under nitrogen atmosphere, and the temperature was then increased. The reaction was initiated at 130°C, and the temperature was then increased to 180°C over 100 minutes. Thereafter, the reaction solution was further stirred for 20 minutes, and it was then cooled to room temperature. As a result of the analysis by HPLC, the conversion rate was 100.0%.

### (Example 17) Synthesis of (R)-3-methylhexanoic acid (addition of copper (I) oxide)

2.00 g (11.5. mmol) of (R)-(1-methylbutyl)malonic acid, 82.8 mg (0.58 mmol) of copper (I) oxide, and 20 ml of acetonitrile were added to a 100-ml flask under nitrogen atmosphere, and the mixture was heated to reflux. After the reaction for 6 hours, the reaction solution was cooled to room temperature. As a result of the analysis by HPLC, the conversion rate was 64.03%.

### (Example 18) Synthesis of (R)-3-methylhexanoic acid (no additives)

3.00 g (17.2 mmol) of (R)-(1-methylbutyl)malonic acid was added to an eggplant-shaped flask under nitrogen atmosphere, and the temperature was then increased. The reaction was initiated at 130°C, and the temperature was then increased to 180°C over 75 minutes Thereafer, the reaction solution was further stirred for 15 minutes, and it was then cooled to room temperature. As a result of the analysis by HPLC, the conversion rate was 100.0%.

The results of the aforementioned Examples 9 to 18 are shown in the following Table 5.

**Table 5**

| Example | Additive | Amount | Solvent | Amount | Reaction temperarure/°C | Time | Invert ratio |
|---|---|---|---|---|---|---|---|
| 9 | - | - | Pyridine | 2 VR | 90 to 130 | 2.4 h | 100% |
| 10 | - | - | DMSO | 2 VR | 100 to 140 | 1.6h | 100% |
| 11 | Pyridine | 0.2 MR | - | - | 100 to 150 | 1.8h | 100% |
| 12 | DABCO | 0.2 MR | - | - | 100 to 140 | 1.6h | 100% |
| 13 | DBU | 0.2 MR | - | - | 100 to 140 | 1.2h | 100% |
| 14 | Ac₂O | 0.5 MR | Pyridine | 2 VR | rt to 40 | 3 h | 100% |
| 15 | H₂SO₄ | 0.1 MR | - | - | 130 to 180 | 2 h | 100% |
| 16 | Fe₃O₄ | 2 wt% | - | - | 130 to 180 | 2 h | 100% |
| 17 | Cu₂O | 0.05 MR | CH₃CN | 10 VR | 80 | 6 h | 64% |
| 18 | - | - | - | - | 130 to 180 | 1.5 h | 100% |

### (Example 19) Synthesis of (S)-2-methanesulfonyloxy pentane

417 g of an ethyl acetate solution that contained 100 g (1.13 mol; 100%ee) of (S)-2-pentanol, and 149 g (1.47 mol) of triethylamine, were added to a 2-L separable flask. The obtained solution was cooled to 5°C, and 156 g (1.36 ml) of methanesulfonyl chloride was then added dropwise thereto. Thereafter, the obtained mixture was stirred at room temperature for 1 hour, and 440 g of water was added to the reaction solution to terminate the reaction. An aqueous layer was eliminated, and 267 g of a 10% sodium chloride solution was then added to an organic layer. Thereafter, the pH of the aqueous layer was converted to be neutral by addition of a 5% sodium hydrogencarbonate aqueous solution. The aqueous layer was eliminated, and the solvent was then distilled away. Thereafter, addition of toluene and concentration were repeated twice, so as to obtain 191 g (light brown oil; chemical purity: 93.5%; 1.07 mol; yield: 94.6%) of crude (S)-2-methanesulfonyloxy pentane.

### (Example 20) Synthesis of (R)-diethyl(1-methylbutyl)malonate

71.7 g (1.79 mol) of 60% sodium hydride in mineral oil and 605 g of THF were added to a 4000-ml separable flask. 287 g (1.79 mmol) of diethyl malonate and 28.4 g of THF were added dropwise to the reaction solution at a temperature between 25°C and 40°C, and the obtained mixture was then washed with 28.4 g of THF. The reaction solution was heated to a temperature between 65°C and 70°C, and 266 g (purity: 80.1%; 1.28 mol) of crude (S)-2-methanesulfonyloxy pentane and 28.4 g of THF were added thereto. Thereafter, the obtained mixture was reacted under reflux for 6 hour, and 638 ml of water was added thereto at room temperature. Thereafter, concentrated hydrochloric acid was added to the mixture, so as to control the pH of the mixture at pH 6 to 7. An aqueous layer was eliminated, and the solvent was then distilled away, so as to obtain 443 g (colorless oil; purity: 59.9%; 1.15 mol; yield: 90.5%) of crude (R)-diethyl(1-me3thylbutyl)malonate.

### (Example 21) Synthesis of (R)-(1-methylbutyl)malonic acid

439 g (purity: 59.9%; 1.14 mmol) of crude (R)-diethyl(1-methylbutyl)malonate, 6,650 g (4.04 mol) of a 25% sodium hydroxide aqueous solution, and 797 ml of water were added to a 4000-ml separable flask. The obtained mixture was reacted at a temperature between 65°C and 70°C for 6 hours, and it was then cooled. The reaction solution was once removed from the flask, and 421 g (4.05 mol) of concentrated hydrochloric acid was added to the flask. Thereafter, the reaction solution was added dropwise thereto. The pH of the mixed solution was pH 1.1. The mixed solution was then extracted with 717 g of ethyl acetate. An organic layer was washed with 532 ml of 0.26 M hydrochloric acid, and the solvent was then distilled away, followed by substitution with toluene. The resultant was crystallized from 974 g of toluene, so as to obtain 170 g (white platy crystal; purity: 95.3 %; 0.931 mmol; yield: 81.7%) of (R)-(1-methylbutyl)malonic acid. As a result of chiral analysis, it was found that the optical purity thereof was 99.7%ee.

### (Example 22) Synthesis of (R)-3-methylhexanoic acid

89 g of pyridine was added to a 1-L eggplant-shaped flask under nitrogen atmosphere, and the temperature was then increased to 110°C. Thereafter, a solution obtained by dissolving 140 g (766 mmol; purity: 95.4%) of (R)-(1-methylbutyl)malonic acid in 147 g of pyridine was added dropwise thereto over 5 hours. The obtained mixture was further stirred for 1 hour, and it was then cooled to room temperature, so as to obtain 329 g of a pyridine solution of (R)-3-methylhexanoic acid. As a result of the analysis by HPLC, it was found that the conversion rate was 100%, and that the yield was quantitative.

754 g of a pyridine solution that contained 169 g (1.29 mmol) of (R)-3-methylhexanoic acid obtained by the reaction was subjected to vacuum distillation at approximately 50 Torr, so as to eliminate pyridine, thereby obtaining 197 g of crude (R)-3-methylhexanoic acid. 190 g of the crude (R)-3-methylhexanoic acid was rectified at approximately 10 Torr, so as to obtain 152 g (colorless oil; 1.17 mol; yield: 90%; purity: 99.3%) of (R)-3-methylhexanoic acid. As a result of chiral analysis, it was found that the optical purity thereof was 99.5%ee.

### INDUSTRIAL APPLICABILITY

According to the method of the present invention, (S)-2-pentanol or (S)-2-hexanol that is an industrially useful compound as an intermediate material for pharmaceuticals, agrichemicals or the like, can be obtained at high optical purity and at a high concentration.

According to the present invention, optically active 1-methylalkyl malonic acid and optically active 3-methyl carboxylic acid that are useful as pharmaceutical or agrichemical intermediates can be obtained at high optical purity by an inexpensive and efficient, industrial production method.

The present invention therefor provides:
[1] A method of producing (S)-2-pentanol which comprises allowing microorganisms or transformed cells, a product obtained by treating said microorganisms or cells, a culture solution of said microorganisms or cells, and/or a crude purified product or purified product of a carbonyl reductase fraction obtained from said microorganisms or cells, to act on 2-pentanone, wherein when a fresh cell mass of said microorganisms or transformed cells, which has not been pretreated with a solvent, is allowed to act on 2-pentanone, (S)-2-pentanol having an optical purity of 95% e.e. or greater can be generated, and the productivity thereof is 1 mg or more of (S)-2-pentanol/g of dry cell mass weight/hour.
[2] A method of producing (S)-2-hexanol which comprises allowing microorganisms or transformed cells, a product obtained by treating said microorganisms or cells, a culture solution of said microorganisms or cells, and/or a crude purified product or purified product of a carbonyl reductase fraction obtained from said microorganisms or cells, to act on 2-hexanone, wherein when a fresh cell mass of said microorganisms or transformed cells, which has not been pretreated with a solvent, is allowed to act on 2-hexanone, (S)-2-hexanol having an optical purity of 95% e.e. or greater can be generated, and the productivity thereof is 1 mg or more of (S)-2-hexanol/g of dry cell weight/hour.
[3] A method for producing (S)-2-pentanol or (S)-2-hexanol having high optical purity, wherein microorganisms selected from the group consisting of genus Brettanomyces, genus Candida, genus Hortaea, genus Issatchenkia, genus Lodderomyces, genus Pichia, genus Rhodotorula, genus Arthrobacter, genus Brevibacterium, genus Crutobacterium, genus Geobacillus, genus Microbacterium, genus Ochrobactrum, genus Paracoccus, genus Rhizobium, and genus Rhodococcus, a product obtained by treating said microorganism, a culture solution of said microorganisms, and/or a crude purified product or purified product of a carbonyl reductase fraction obtained from said microorganisms, are allowed to act on 2-pentanone or 2-hexanone, so as to generate (S)-2-pentanol or (S)-2-hexanol.
[4] A method for producing (S)-2-pentanol or (S)-2-hexanol having high optical purity, wherein transformed cells wherein DNA encoding carbonyl reductase obtained from microorganisms selected from the group consisting of genus Brettanomyces, genus Candida, genus Hortaea, genus Issatchenkia, genus Lodderomyces, genus Pichia, genus Rhodotorula, genus Arthrobacter, genus Brevibacterium, genus Crutobacterium, genus Geobacillus, genus Microbacterium, genus Ochrobactrum, genus Paracoccus, genus Rhizobium, and genus Rhodococcus, has been allowed to express, a product obtained by treating said cells, a culture solution of said cells, and/or a crude purified product or purified product of a carbonyl reductase fraction obtained from said cells, are allowed to act on 2-pentanone or 2-hexanone, so as to generate (S)-2-pentanol or (S)-2-hexanol.
[5] The production method according to [3] or [4], wherein the microorganisms are selected from the group consisting of *Brettanomyces bruxellensis, Brettanomyces anomallus, Candida famata, Candida krusei, Candida maltosa, Candida tropicalis, Candida zeylanoides, Hortaea werneckii, Issatchenkia scutulata, Lodderomyces elongisporus, Pichia angusta, Pichia besseyi, Pichia cactophila, Pichia segobiensis, Pichia spartinae, Pichia trehalophila, Rhodotorula minuta, Arthrobacter oxydans, Arthrobacter polychromogenes,* Arthrobacter sp., *Arthrobacter sulfurous, Brevibacterium butanicum, Curtobacterium flaccumfaciens, Geobacillus stearothermophilus, Microbacterium keratanolyticum, Microbacterium saperdae,* Microbacterium sp., *Microbacterium testaceum, Ochrobactrum anthropi,* Ochrobactrum sp. (*Pseudomonas ovalis), Pracoccus denitrificans, Rhizobium radiobacter,* and Rhodococcus sp. (*Corynebacterium hydrocarboclastum*)*.*
[6] A method for producing (S)-2-pentanol or (S)-2-hexanol having high optical purity, wherein transformed cells, wherein DNA described in any one of the following (A) to (F) has been allowed to express, a product obtained by treating said cells, and/or a culture solution of said cells, are allowed to act on 2-pentanone or 2-hexanone, so as to generate (S)-2-pentanol or (s)-2-hexanol:
   (A) DNA encoding a protein having the amino acid sequence shown in SEQ ID NO: 1;
   (B) DNA encoding a protein, which has an amino acid sequence comprising a deletion, addition, or substitution of one or several amino acids with respect to the amino acid sequence shown in SEQ ID NO: 1, and which has ability to reduce a carbonyl group to synthesize optically active alcohol;
   (C) DNA encoding a protein, which has an amino acid sequence showing homology of 50% or more with the amino acid sequence shown in SEQ ID NO: 1, and which has ability to reduce a carbonyl group to synthesize optically active alcohol;
   (D) DNA having the nucleotide sequence shown in SEQ ID NO: 2;
   (E) DNA having a nucleotide sequence, which comprises a deletion, addition, or substitution of one or several nucleotides with respect to the nucleotide sequence shown in SEQ ID NO: 2, and which encodes a protein having ability to reduce a carbonyl group to synthesize Optically active alcohol; and
   (F) DNA having a nucleotide sequence, which hybridizes with the nucleotide sequence shown in SEQ ID NO: 2 or a complementary sequence thereof under stringent conditions, and which encode a protein having ability to reduce a carbonyl group to synthesize optically active alcohol.
[7] A method for producing (R)- or (S)-3-methyl carboxylic acid represented by the following formula (5): 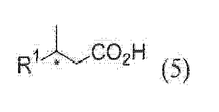 wherein R¹ represents an alkyl group containing 3 to 5 carbon atoms, and * represents an asymmetric carbon,
   which comprises decarboxylating (R)- or (S)-1-methylalkyl malonic acid having optical activity represented by the following formula (1) in the presence of a highly polar solvent and/or an additive for promoting decarboxylation: 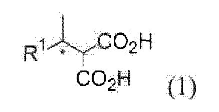 wherein R¹ has the same definition as described above, and * represents an asymmetric carbon.
[8] A method for producing (R)- or (S)-1-methylalkyl malonic acid represented by the following formula (1): 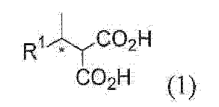 wherein R¹ represents an alkyl group containing 3 to 5 carbon atoms, and * represents an asymmetric carbon,
   which comprises allowing Optically active alcohol represented by the following formula (2) to react with a sulfonylation agent: 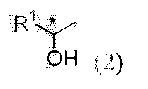 wherein R¹ has the same definition as described above, and * represents an asymmetric carbon, so as to obtain an optically active compound represented by the following formula (3): 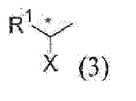 wherein R¹ has the definition as described above, X represents a sulfonyloxy group, and * represents an asymmetric carbon;
   allowing the optically active compound to react with a carbon nucleophile represented by the following formula (9) in the presence of a base: 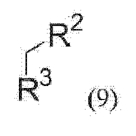 wherein each of R² and R³ independently represents an ester group, a carboxyl group, or a cyano group, wherein R² and R³ may together form a cyclic structure, so as to obtain an optically active compound represented by the following formula (4): 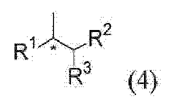 wherein R¹, R², and R³ have the same definitions as described above, and * represents an asymmetric carbon, and
   hydrolyzing the obtained optically active compound.
[9] (R)-1-methylalkyl malonic acid or (S)-1-methylalkyl malonic acid having an optical purity of 90%ee or greater, which is represented by the following formula (1)_{:} 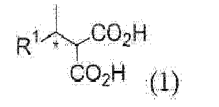 wherein R¹ represents an alkyl group containing 3 to 5 carbon atoms, and * represents an asymmetric carbon.
[10] The (R)-1-methylalkyl malonic acid or (S)-1-niethylalkyl malonic acid according to [9], wherein R¹ represents an n-propyl group or an n-butyl group.
[11] A method for producing an optically active substance represented by the following formula (6): 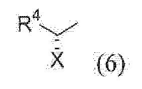 wherein R⁴ represents an n-propyl group, and X represents a sulfonyloxy group,
   which comprises: allowing microorganisms or transformed cells containing a carbonyl reductase having activity to react with 2-pentanone to generate (S)-2-pentanol, wherein it is able to generate (S)-2-pentanol having an optical purity of 95% e.e. or greater when the fresh cell mass thereof, which has not been pretreated with a solvent, is allowed to act on 2-pentanone, and the productivity thereof is 10 mg or more of (S)-2-pentanol/g of dry cell mass weight/hour, a product obtained by treating said microorganisms or cells, a culture solution of said microorganisms or cells, and/or a crude purified product or purified product of a carbonyl reductase fraction obtained from said microorganisms or cells, to act on 2-pentanone, so as to convert it to (S)-2-pentanol; and allowing the obtained (S)-2-pentanol to react with a sulfonylation agent, so as to convert it to the optically active substance represented by the above formula (6).
[12] A method for producing an optically active substance represented by the following formula (6): 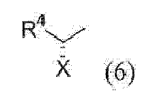 wherein R⁴ represents an n-butyl group, and X represents a sulfonyloxy group,
   which comprises: allowing microorganisms or transformed cells containing a carbonyl reductase having activity to react with 2-hexanone to generate (S)-2-hexanol, wherein it is able to generate (S)-2-hexanol having an optical purity of 95% e.e. or greater when the fresh cell mass thereof, which has not been pretreated with a solvent, is allowed to act on 2-hexanone, and the productivity thereof is 10 mg or more of (S)-2-hexanol/g of dry cell mass weight/hour, a product obtained by treating said microorganisms or cells, a culture solution of said microorganisms or cells, and/or a crude purified product or purified product of a carbonyl reductase fraction obtained from said microorganisms or cells, to act on 2-hexanone, so as to convert it to (S)-2-hexanol; and allowing the obtained (S)-2-hexanol to react with a sulfonylation agent, so as to convert it to the optically active substance represented by the above formula (6).
[13] The method according to [11] or [12], which further comprises a step of allowing the optically active substance represented by formula (6) to react with a carbon nucleophile represented by the following formula (9) in the presence of a base: 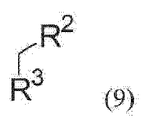 wherein each of R² and R³ independently represents an ester group, a carboxyl group, or a cyano group, wherein R² and R³ may together form a cyclic structure, so as to convert it to an optically active compound represented by the following formula (7): 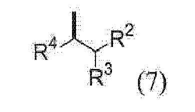 wherein R² and R³ have the same definitions as described above, and R⁴ represents an n-propyl group or an n-butyl group.
[14] A method for producing (R)-1-methylbutyl malonic acid or (R)-1-methylpentyl malonic acid, which comprises; allowing the optically active substance represented by formula (6) obtained by the method according to [11] or [12] to react with a carbon nucleophile represented by the following formula (9) in the presence of a base: 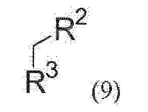 wherein each of R² and R³ independently represents an ester group, a carboxyl group, or a cyano group, wherein R² and R³ may together form a cyclic structure, so as to convert it to an optically active compound represented by the following formula (7): 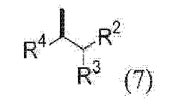 wherein R² and R³ have the same definitions as described above, and R⁴ represents an n-propyl group or an n-butyl group, and
   hydrolyzing the obtained optically active compound, so as to convert it to (R)-1-methylbutyl malonic acid or (R)-1-methylpentyl malonic acid represented by the following formula (8): 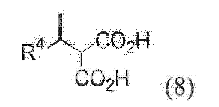 wherein R⁴ has the same definition as described above.
[15] A method for producing (R)-3-methyl hexanoic acid or (R)-3-methyl heptanoic acid, which comprises allowing the optically active substance represented by formula (6) obtained by the method according to [11] or [12] to react with a carbon nucleophile represented by the following formula (9) in the presence of a base: 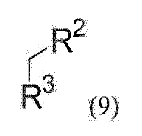 wherein each of R² and R³ independently represents an ester group, a carboxyl group, or a cyano group, wherein R² and R³ may together form a cyclic structure, so as to convert it to an optically active compound represented by the following formula (7): 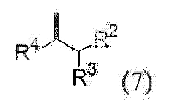 wherein R² and R³ have the same definitions as described above, and R⁴ represents an n-propyl group or an n-butyl group,
   hydrolyzing the obtained optically active compound, so as to convert it to (R)-1-methylbutyl malonic acid or (R)-1-methylpentyl malonic acid represented by the following formula (8): 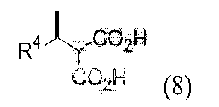 wherein R⁴ has the same definition as described above, and
   decarboxylating the obtained (R)-1-methylbutyl malonic acid or (R)-1-methylpentyl malonic acid.

## Claims

1. A method for producing (R)- or (S)-3-methyl carboxylic acid represented by the following formula (5): 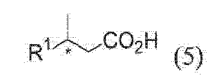 wherein R¹ represents an alkyl group containing 3 to 5 carbon atoms, and * represents an asymmetric carbon,
which comprises decarboxylating (R)- or (S)-1-methylalkyl malonic acid having optical activity represented by the following formula (1) in the presence of a highly polar solvent and/or an additive for promoting decarboxylation: 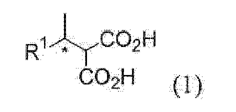 wherein R¹ has the same definition as described above, and * represents an asymmetric carbon.


SEQUENCE LISTING

<110> API Corporation

<120> Process for producing optically active alcohol and carboxylic
      acid

<130> P21876bEP

<140> 05 710 145.3
<141> 2005-02-04

<150> JP20040027815
<151> 2004-02-04

<150> JP20040147023
<151> 2004-04-13

<160> 13

<170> PatentIn version 3.3

<210> 1
<211> 345
<212> PRT
<213> Issatchenkia scutulata

<400> 1

Met Ser Asn Lys Thr Val Leu Val Thr Gly Ala Thr Gly Phe Ile Ala
1               5                   10                  15

Leu His Ile Ile Asp Asn Leu Leu Ser Lys Gly Tyr Ser Val Ile Gly
            20                  25                  30

Thr Ala Arg Ser Gln Ser Lys Tyr Gln Pro Ile Leu Asp Ala Phe Lys
        35                  40                  45

Lys Lys Tyr Pro Asp Ala Asn Leu Thr Phe Glu Val Val Pro Asp Ile
    50                  55                  60

Ser Thr Glu Asn Ala Phe Asp Asp Val Leu Lys Lys His Pro Glu Ile
65                  70                  75                  80

Thr Ala Val Leu His Thr Ala Ser Pro Phe Ser Phe Gly Leu Asn Lys
                85                  90                  95

Asp Leu Lys Glu Ala Tyr Leu Lys Pro Ala Val Asp Gly Thr Leu Asn
            100                 105                 110

Ile Leu Lys Ala Ile Glu Lys Tyr Ala Pro Gln Val Thr Lys Val Val
        115                 120                 125

Ile Thr Ser Ser Tyr Ala Ala Ile Met Thr Gly Asn Pro Ser His Val
    130                 135                 140

His Thr Ser Glu Thr Trp Asn Pro Ile Asn Trp Glu Asn Asp Val Lys
145                 150                 155                 160

Asn Glu Tyr Phe Ala Tyr Ile Ala Ser Lys Thr Tyr Ala Glu Lys Ala
                165                 170                 175

Ala Arg Asp Phe Val Lys Glu His Lys Val Asn Phe Lys Leu Ala Thr
            180                 185                 190

Val Asn Pro Pro Tyr Val Leu Gly Pro Gln Leu Phe Asp Phe Ser Val
        195                 200                 205

Gly Pro Val Leu Asn Thr Ser Asn Gln Leu Ile Thr Asp Ala Thr Lys
    210                 215                 220

Ile Asp Lys Asn Ser Thr Lys Pro Glu Leu Gly Thr Pro Ala Leu Ala
225                 230                 235                 240

Val Asp Val Arg Asp Val Ala Ala Phe His Val Leu Pro Leu Glu Asp
                245                 250                 255

Asp Lys Val Ala Ser Glu Arg Leu Phe Ile Val Ala Gly Pro Ala Val
            260                 265                 270

Val Gln Thr Phe Leu Asn Ile Ile Asn Glu Asn Ile Pro Glu Leu Lys
        275                 280                 285

Gly Lys Val Ala Leu Gly Asp Pro Ala Ser Glu Lys Glu Leu Ile Glu
    290                 295                 300

Lys His Thr Asp Lys Tyr Asp Leu Thr Asn Leu His Asn Val Ile Gly
305                 310                 315                 320

Lys Tyr Asp Phe Ile Pro Val Glu Lys Ser Val Val Asp Val Leu Glu
                325                 330                 335

Gln Tyr Tyr Lys Ile Asn Lys Ile Asp
            340                 345

<210>  2
<211>  1038
<212>  DNA
<213>  Issatchenkia scutulata

<400>  2
atgtcgaaca aaacagttct agtcaccggg gctaccggtt ttattgcact acacatcatt      60

gataatttat tgtctaaggg ttattccgtt attggtacag ctagatccca atctaaatat     120

caaccaatcc ttgatgcttt caagaaaaaa taccctgatg caaatttgac ttttgaagtt     180

gtccctgaca tctccactga aaacgcattc gatgatgttt tgaagaagca tccagaaatt     240

actgctgtcc ttcacacagc atctccattc tcttttggtt tgaacaagga tctgaaggaa     300

gcatatttga agcctgccgt tgatggtact ttgaatattc tcaaggcaat tgagaagtat     360

gcaccacagg ttactaaagt tgttatcaca tcttcttatg ctgcaattat gacaggtaat     420

ccaagtcatg tccacaccag tgaaacctgg aacccaatta attgggaaaa cgatgtgaag 480

aatgaatact ttgcatatat tgcctccaag acgtatgctg aaaaagctgc gagagatttt 540

gtcaaggagc ataaggtcaa tttcaagtta gcaactgtta acccaccata cgttctgggt 600

ccacaattat ttgacttctc agttggtcca gtcttgaaca cttccaacca attgatcacg 660

gatgcgacta aaattgataa gaactctact aagccggaat taggtacacc agctttagca 720

gtcgatgtta gagatgttgc tgcgttccat gttttaccat tggaagatga taaagttgca 780

agtgaaagat tatttattgt tgctggtcca gcagttgttc aaacattctt aaacatcatc 840

aacgagaaca ttccagaact taaaggtaag gttgccctag gagatccagc ttcagagaag 900

gagttgattg aaaagcacac agataagtat gatttgacaa atcttcacaa cgttattggt 960

aaatatgatt tcattccagt tgaaaagtcc gttgtcgacg tcttagaaca atattacaaa 1020

atcaataaaa ttgattag 1038

<210> 3
<211> 344
<212> PRT
<213> Saccharomyces cerevisiae

<400> 3

Met Ser Asn Thr Val Leu Val Ser Gly Ala Ser Gly Phe Ile Ala Leu
1               5                   10                  15

His Ile Leu Ser Gln Leu Leu Lys Gln Asp Tyr Lys Val Ile Gly Thr
            20                  25                  30

Val Arg Ser His Glu Lys Glu Ala Lys Leu Leu Arg Gln Phe Gln His
        35                  40                  45

Asn Pro Asn Leu Thr Leu Glu Ile Val Pro Asp Ile Ser His Pro Asn
    50                  55                  60

Ala Phe Asp Lys Val Leu Gln Lys Arg Gly Arg Glu Ile Arg Tyr Val
65                  70                  75                  80

Leu His Thr Ala Ser Pro Phe His Tyr Asp Thr Thr Glu Tyr Glu Lys
                85                  90                  95

Asp Leu Leu Ile Pro Ala Leu Glu Gly Thr Lys Asn Ile Leu Asn Ser
            100                 105                 110

Ile Lys Lys Tyr Ala Ala Asp Thr Val Glu Arg Val Val Val Thr Ser
        115                 120                 125

Ser Cys Thr Ala Ile Ile Thr Leu Ala Lys Met Asp Asp Pro Ser Val
    130                 135                 140

Val Phe Thr Glu Glu Ser Trp Asn Glu Ala Thr Trp Glu Ser Cys Gln

145                 150                 155                 160

Ile Asp Gly Ile Asn Ala Tyr Phe Ala Ser Lys Lys Phe Ala Glu Lys
                165                 170                 175

Ala Ala Trp Glu Phe Thr Lys Glu Asn Glu Asp His Ile Lys Phe Lys
            180                 185                 190

Leu Thr Thr Val Asn Pro Ser Leu Leu Phe Gly Pro Gln Leu Phe Asp
        195                 200                 205

Glu Asp Val His Gly His Leu Asn Thr Ser Cys Glu Met Ile Asn Gly
    210                 215                 220

Leu Ile His Thr Pro Val Asn Ala Ser Val Pro Asp Phe His Ser Ile
225                 230                 235                 240

Phe Ile Asp Val Arg Asp Val Ala Leu Ala His Leu Tyr Ala Phe Gln
                245                 250                 255

Lys Glu Asn Thr Ala Gly Lys Arg Leu Val Val Thr Asn Gly Lys Phe
            260                 265                 270

Gly Asn Gln Asp Ile Leu Asp Ile Leu Asn Glu Asp Phe Pro Gln Leu
        275                 280                 285

Arg Gly Leu Ile Pro Leu Gly Lys Pro Gly Thr Gly Asp Gln Val Ile
    290                 295                 300

Asp Arg Gly Ser Thr Thr Asp Asn Ser Ala Thr Arg Lys Ile Leu Gly
305                 310                 315                 320

Phe Glu Phe Arg Ser Leu His Glu Ser Val His Asp Thr Ala Ala Gln
                325                 330                 335

Ile Leu Lys Lys Glu Asn Arg Leu
            340

<210>  4
<211>  18
<212>  PRT
<213>  Issatchenkia scutulata

<400>  4

Arg Asn Lys Thr Val Leu Val Thr Gly Ala Thr Gly Phe Ile Ala Leu
1               5                   10                  15

Asp Ile

<210>  5
<211>  15

<212>  PRT
<213>  Issatchenkia scutulata

<400>  5

Val Val Ile Thr Ser Ser Tyr Ala Ala Ile Met Thr Gly Asn Pro
1               5                   10                  15


<210>  6
<211>  23
<212>  DNA
<213>  artificial

<220>
<223>  synthetic DNA


<220>
<221>  misc_feature
<222>  (3)..(3)
<223>  n is inosine

<220>
<221>  misc_feature
<222>  (6)..(6)
<223>  n is inosine

<220>
<221>  misc_feature
<222>  (15)..(15)
<223>  n is inosine

<220>
<221>  misc_feature
<222>  (18)..(18)
<223>  n is inosine

<400>  6
acnggnttya thgcnytnga yat                                               23


<210>  7
<211>  26
<212>  DNA
<213>  Artificial

<220>
<223>  synthetic DNA


<220>
<221>  misc_feature
<222>  (6)..(6)
<223>  n is inosine

<220>
<221>  misc_feature
<222>  (9)..(9)
<223>  n is inosine

<220>
<221>  misc_feature
<222>  (18)..(18)
<223>  n is inosine

<220>
<221>  misc_feature

<222>  (21)..(21)
<223>  n is inosine

<400>  7
ggrttnccng tcatdatngc ngcrta                                          26


<210>  8
<211>  341
<212>  DNA
<213>  Issatchenkia scutulata

<400>  8
cattgataat ttattgtcta agggttattc cgttattggt acagctagat cccaatctaa      60

atatcaacca atccttgatg ctttcaagaa aaaataccct gatgcaaatt tgacttttga     120

agttgtccct gacatctcca ctgaaaacgc attcgatgat gttttgaaga agcatccaga     180

aattactgct gtccttcaca cagcatctcc attctctttt ggtttgaaca aggatctgaa     240

ggaagcatat ttgaagcctg ccgttgatgg tactttgaat attctcaagg caattgagaa     300

gtatgcacca caggttacta aagttgttat cacatcttct t                         341


<210>  9
<211>  28
<212>  DNA
<213>  artificial

<220>
<223>  synthetic DNA

<400>  9
agggttattc cgttattggt acagctag                                        28


<210>  10
<211>  31
<212>  DNA
<213>  artificial

<220>
<223>  synthetic DNA

<400>  10
gagaatggag atgctgtgtg aaggacagca g                                    31


<210>  11
<211>  1212
<212>  DNA
<213>  Issatchenkia scutulata

<400>  11
tcatgacctg tccactgata gcatcatacc aaacatattc agtatattgt aacaatgtcg      60

aacaaaacag ttctagtcac cggggctacc ggttttattg cactacacat cattgataat     120

ttattgtcta agggttattc cgttattggt acagctagat cccaatctaa atatcaacca     180

atccttgatg ctttcaagaa aaaataccct gatgcaaatt tgacttttga agttgtccct     240

gacatctcca ctgaaaacgc attcgatgat gttttgaaga agcatccaga aattactgct     300

gtccttcaca cagcatctcc attctctttt ggtttgaaca aggatctgaa ggaagcatat     360

ttgaagcctg ccgttgatgg tactttgaat attctcaagg caattgagaa gtatgcacca 420

caggttacta aagttgttat cacatcttct tatgctgcaa ttatgacagg taatccaagt 480

catgtccaca ccagtgaaac ctggaaccca attaattggg aaaacgatgt gaagaatgaa 540

tactttgcat atattgcctc caagacgtat gctgaaaaag ctgcgagaga ttttgtcaag 600

gagcataagg tcaatttcaa gttagcaact gttaacccac catacgttct gggtccacaa 660

ttatttgact tctcagttgg tccagtcttg aacacttcca accaattgat cacggatgcg 720

actaaaattg ataagaactc tactaagccg gaattaggta caccagcttt agcagtcgat 780

gttagagatg ttgctgcgtt ccatgtttta ccattggaag atgataaagt tgcaagtgaa 840

agattattta ttgttgctgg tccagcagtt gttcaaacat tcttaaacat catcaacgag 900

aacattccag aacttaaagg taaggttgcc ctaggagatc cagcttcaga gaaggagttg 960

attgaaaagc acacagataa gtatgatttg acaaatcttc acaacgttat tggtaaatat 1020

gatttcattc cagttgaaaa gtccgttgtc gacgtcttag aacaatatta caaaatcaat 1080

aaaattgatt agtttatata gaaaatttta tagctaaagg ccgaatcaac ttctttcttc 1140

ctcttcaaaa aaaaaaaaaa aaaaaaaaaa aaaaaaaaaa aaaaaaaaaa aaaaaaaaaa 1200

aaaaaaaaaa aa 1212

<210> 12
<211> 35
<212> DNA
<213> Artificial

<220>
<223> synthetic DNA

<400> 12
cggaattcat gtcgaacaaa acagttctag tcacc 35

<210> 13
<211> 38
<212> DNA
<213> Artificial

<220>
<223> synthetic DNA

<400> 13
gctctagatt aatcaatttt attgattttg taatattg 38